(19) Europäisches Patentamt
European Patent Office
Office européen des brevets

(11) Veröffentlichungsnummer: **0 034 205**
**A1**

(12) # EUROPÄISCHE PATENTANMELDUNG

(21) Anmeldenummer: **80106946.9**

(22) Anmeldetag: **11.11.80**

(51) Int. Cl.³: **C 07 C 147/00**
C 07 D 333/36, A 01 N 41/10
A 01 N 43/10

(30) Priorität: **21.11.79 DE 2946910**

(43) Veröffentlichungstag der Anmeldung: **26.08.81 Patentblatt 81/34**

(84) Benannte Vertragsstaaten: **AT BE CH DE FR GB IT LI LU NL SE**

(71) Anmelder: **HOECHST AKTIENGESELLSCHAFT**
**Postfach 80 03 20**
**D-6230 Frankfurt/Main 80(DE)**

(72) Erfinder: **Koch, Manfred, Dr.**
**Kreuzheck 2**
**D-6239 Eppstein/Taunus(DE)**

(72) Erfinder: **Mildenberger, Hilmar, Dr.**
**Fasanenstrasse 24**
**D-6233 Kelkheim (Taunus)(DE)**

(72) Erfinder: **Sachse, Burkhard, Dr.**
**An der Ziegelei 30**
**D-6233 Kelkheim (Taunus)(DE)**

(54) **Fungizide Sulfonyl- bzw. Sulfinylacetanilide, Verfahren zu ihrer Herstellung und diese enthaltende Schädlingsbekämpfungsmittel.**

(57) Gegenstand der Erfindung sind neue Sulfonyl -bzw. Sulfinyl-acetanilide der allgemeinen Formel I,

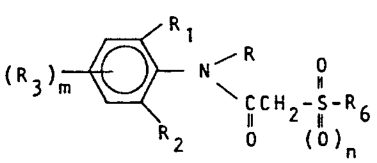 (1)

worin
R = 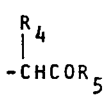 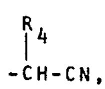 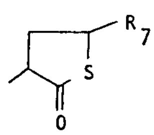,

$R_1$ = $(C_1-C_4)$-Alkyl, Halogen, $(C_1-C_4)$-Alkoxy,$(C_1-C_4)$-Alkylthio, $(C_2-C_4)$-Alkenyl,

$R_2$ = Wasserstoff, $(C_1-C_4)$-Alkyl, Halogen,
$R_3$ = $(C_1-C_4)$-Alkyl, Halogen,
$R_4$, $R_7$ = Wasserstoff, Methyl,
$R_5$ = Hydroxy, $(C_1-C_4)$-Alkoxy, $(C_1-C_3)$-Alkylthio, $(C_1-C_2)$-Alkoxyäthoxy, Amino, $(C_1-C_2)$-Alkylamino, Di-$(C_1-C_2)$-alkyl-amino, O-Kat, wobei Kat für ein Kationäquivalent einer anorganischen oder organischen Base steht,
$R_6$ = $(C_1-C_8)$-Alkyl, $(C_1-C_4)$-Alkoxy-$(C_1-C_3)$-alkyl, $(C_1-C_2)$-Alkylthio-$(C_1-C_3)$-alkyl, $(C_1-C_2)$-Alkylsulfinyl-$(C_1-C_3)$-alkyl, $(C_1-C_2)$-Alkylsulfonyl-$(C_1-C_3)$-alkyl, $(C_3-C_6)$-Cycloalkyl, $(C_1-C_4)$-Halogenalkyl, $(C_1-C_4)$-Hydroxyalkyl, $(C_1-C_2)$-Alkoxycarbonyl-$(C_1-C_2)$-alkyl, $(C_3-C_4)$-Alkenyl, $(C_3-C_4)$-Alkinyl, gegebenenfalls substituiertes Benzyl, gegebenenfalls substituiertes Phenyl,
n = 0, 1 und m = 0, 1, 2 bedeuten,
ferner Verfahren zu deren Herstellung sowie deren Verwendung als Schädlingsbekämpfungsmittel.

EP 0 034 205 A1

0034205

HOECHST AKTIENGESELLSCHAFT HOE 79/F 314 Dr.GM/Wa

# Fungizide Sulfonyl- bzw. Sulfinylacetanilide, Verfahren zu ihrer Herstellung und diese enthaltende Schädlingsbekämpfungsmittel

Gegenstand der vorliegenden Erfindung sind neue, fungizide Sulfonyl-, bzw. Sulfinylacetanilide, Verfahren zu ihrer Herstellung sowie ihre Verwendung als Schädlingsbekämpfungsmittel, insbesondere im Pflanzenschutz.

Die erfindungsgemäßen Verbindungen besitzen die allgemeine Formel I,

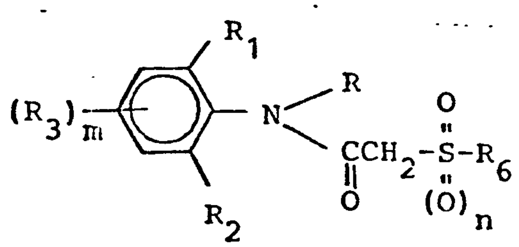

(I)

worin

R = 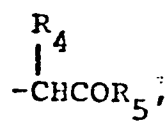, 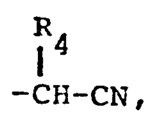, 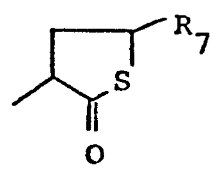,

$R_1$ = $(C_1-C_4)$-Alkyl, Halogen, $(C_1-C_4)$-Alkoxy, $(C_1-C_4)$-Alkylthio, $(C_2-C_4)$-Alkenyl,

$R_2$ = Wasserstoff, $(C_1-C_4)$-Alkyl, Halogen,

$R_3$ = $(C_1-C_4$-Alkyl, Halogen

$R_4$, $R_7$ = Wasserstoff, Methyl,

$R_5$ = Hydroxy, $(C_1-C_4)$-Alkoxy, $(C_1-C_3)$-Alkylthio, $(C_1-C_2)$-Alkoxyäthoxy, Amino, $(C_1-C_2)$-Alkylamino, Di-$(C_1-C_2)$-alkylamino, O-Kat, wobei Kat für ein Kationäquivalent einer anorganischen oder organischen Base steht, vorzugsweise Na, K, $\frac{1}{2}$Ca, Ammonium,

$R_6$ = $(C_1-C_8)$-Alkyl, $(C_1-C_4)$-Alkoxy-$(C_1-C_3)$-alkyl, $(C_1-C_2)$-Alkylthio-$(C_1-C_3)$-alkyl, $(C_1-C_2)$-Alkylsulfinyl-$(C_1-C_3)$-alkyl, $(C_1-C_2)$-Alkylsulfonyl-$(C_1-C_3)$-alkyl, $(C_3-C_6)$-Cycloalkyl, $(C_1-C_4)$-Halogenalkyl, $(C_1-C_4)$-Hydroxyalkyl, $(C_1-C_2)$-Alkoxycarbonyl-$(C_1-C_2)$-alkyl, $(C_3-C_4)$-Alkenyl, $(C_3-C_4)$-

BAD ORIGINAL

Alkinyl, Benzyl, das gegebenenfalls substituiert ist durch ein oder zwei Reste aus der Gruppe $(C_1-C_4)$-Alkyl, Halogen, Nitro; Phenyl, das gegebenenfalls substituiert ist durch ein oder zwei Reste aus der Gruppe $(C_1-C_4)$-Alkyl, Halogen,

n = 0, 1

m = 0, 1, 2

bedeutet.

Bevorzugte Reste in Formel I sind:

R = 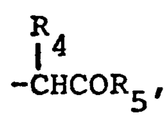, 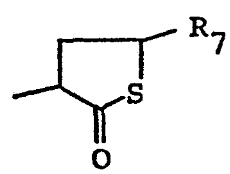,

$R_1$ = $(C_1-C_2)$-Alkyl, $(C_1-C_2)$-Alkoxy, Halogen,

$R_2$ = Wasserstoff, $(C_1-C_2)$-Alkyl, Halogen

$R_3$ = Chlor, Methyl,

$R_4$, $R_7$ = Wasserstoff, Methyl,

$R_5$ = Hydroxy, $(C_1-C_2)$-Alkoxy, $(C_1-C_2)$-Alkylthio, Amino, $(C_1-C_2)$-Alkylamino, Di-$(C_1-C_2)$-alkylamino,

$R_6$ = $(C_1-C_6)$-Alkyl, $(C_1-C_2)$-Alkoxy-$(C_1-C_3)$-alkyl, $(C_3-C_6)$-Cycloalkyl, $(C_1-C_4)$-Halogenalkyl, $(C_1-C_4)$-Hydroxyalkyl, $(C_1-C_2)$-Alkoxycarbonyl-$(C_1-C_2)$-alkyl, $(C_3-C_4)$-Alkenyl, Benzyl oder durch $(C_1-C_4)$-Alkyl, Halogen oder Nitro substituiertes Benzyl,

n = 0, 1

m = 0, 1.

Es ist bekannt, zur Bekämpfung von Fäulniserkrankungen an Pflanzen, welche durch Pilze der Gattung Phythophtora verursacht werden, Fungizide aus der Reihe der Dithiocarbamate, wie zum Beispiel Mancozeb oder Maneb, zinnorganische Verbindungen, wie z.B. Fentinacetat, oder Perchlormethylmerkaptanderivate, wie z.B. Captafol zu verwenden. Mit diesen Wirkstoffen können die oberirdisch, an Blatt- und Stengelwerk angreifenden Phythophtorastämme bekämpft werden, während bodenbürtige Erreger der Gattung Phythophtora und Pythium, welche an den Wurzeln der Pflanzen angreifen, nicht erfaßt werden. Es ist weiter bekannt, daß acylierte Anilinoessig-, bzw. propionsäurederivate, wie sie in den DE-Offenlegungsschriften 23 50 944, 25 13 730, 25 13 789, 25 15 091, 26 43 445 und 26 43 477 beschrieben sind, neben einer herbiziden Wirksamkeit auch eine fungizide Wirkung gegen Pilze der Familie der Oomyceten besitzen.

Es wurde nun überraschenderweise gefunden, daß die erfindungsgemäßen Verbindungen der Formel I eine hervorragende fungizide Wirkung, insbesondere gegen die der Klasse der Phycomyceten angehörenden Oomyceten, wie z.B. Pythium, Phythophtora, Peronospora, Pseudoperonospora und Plasmopara aufweisen.

Damit können Pilze an den verschiedensten Kulturpflanzen, wie z.B. Mais, Reis, Getreide, Zuckerrüben, Gemüse, Gurkengewächse, Kartoffeln, Tomaten, Reben, Hopfen, Tabak, Zitrus-u. Paprikaarten, Zierpflanzen, Kakao, Bananen und Kautschuk bekämpft bzw. gehemmt bzw. ihr Auftreten an diesen Pflanzen ganz verhindert werden. Die Verbindungen der Formel I wirken teilweise systemisch. Sie lassen sich auch als Beizmittel zur Bekämpfung von samenbürtigen Pilzen an Saatgut oder zur Bekämpfung der im Erdboden auftretenden phytopathogenen Pilze einsetzen.

Die Wirkung der erfindungsgemäßen Verbindungen der

Formel I ist u.a. deshalb als überaus überraschend zu bezeichnen, weil entsprechende Alkylthioacetanilide, wie sie in der oben genannten DE-OS 25 15 091 beschrieben sind, gegen Phythophtora, insbesondere gegen bodenbürtige Phythophtoraarten und Pilze der Familie Pythium nur eine sehr geringe fungizide Wirkung aufweisen.

Gegenstand der Erfindung sind daher auch fungizide Mittel, die gekennzeichnet sind durch einen Gehalt an einer Verbindung der Formel I.

Die Herstellung von Verbindungen der Formel I ist nach verschiedenen Methoden möglich und kann z.B. nach den nachstehend aufgeführten Verfahren 1 bis 5 erfolgen.

Die Herstellung von Sulfonylderivaten der allgemeinen Formel I, in der n=1 bedeutet, kann erfolgen:

1. Durch Alkylierung von Sulfinsäuresalzen der allgemeinen Formel II, in der Me ein anorganisches oder organisches Kation, vorzugsweise Natrium, Kalium, Calcium oder Ammonium, bedeutet und $R_6$ die Bedeutung wie in Formel I hat, mit Verbindungen der allgemeinen Formel III,

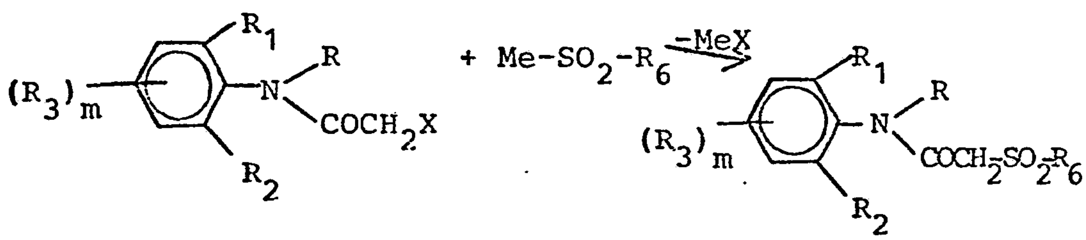


(III) (II) (I)

worin X einen nukleophil substituierbaren Abgangsrest, vorzugsweise aus der Gruppe Halogen, Tosylat oder Mesylat, insbesondere Chlor oder Brom bedeutet, und R, $R_1$, $R_2$, $R_3$ und m die Bedeutungen wie in Formel I haben, oder

2. durch Acylierung von Anilinderivaten der allgemeinen Formel IV, in der R, $R_1$, $R_2$, $R_3$ und m die Bedeutungen wie in Formel I haben, mit Sulfonylacetylhalogeniden der allg. Formel V,

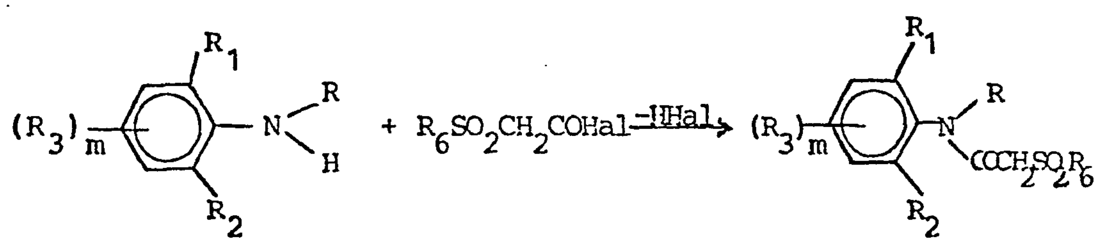


(IV) (V) (I)

worin Hal ein Halogenatom, vorzugsweise Chlor oder Brom, bedeutet und $R_6$ die Bedeutung wie in Formel I hat, oder

3. durch Oxidation von Merkaptoacetaniliden der allgemeinen Formel VI,

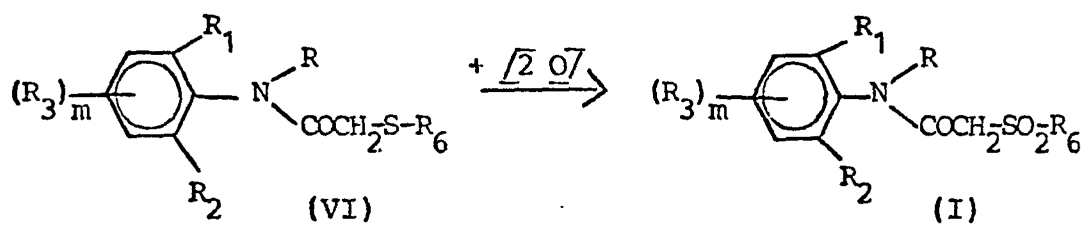


(VI) (I)

in der R, $R_1$, $R_2$, $R_3$, $R_6$ und m die Bedeutungen wie in Formel I haben.

Die Herstellung von Sulfinylderivaten der allgemeinen Formel I, in der n=O bedeutet, kann erfolgen:

4. durch Acylierung von Anilinderivaten der allgemeinen Formel IV, in der R, $R_1$, $R_2$, $R_3$ und m die Bedeutungen wie in Formel I haben, mit Sulfinylacetylhalogeniden der allgemeinen Formel VII,

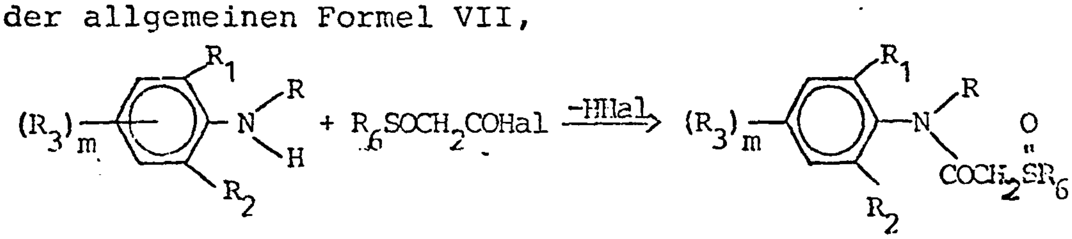


(IV) (VII) (I)

worin Hal ein Halogenatom, vorzugsweise Chlor oder Brom, bedeutet und $R_6$ die Bedeutung wie in Formel I hat, oder

5. durch Oxidation von Merkaptoacetaniliden der allgemeinen Formel VI,

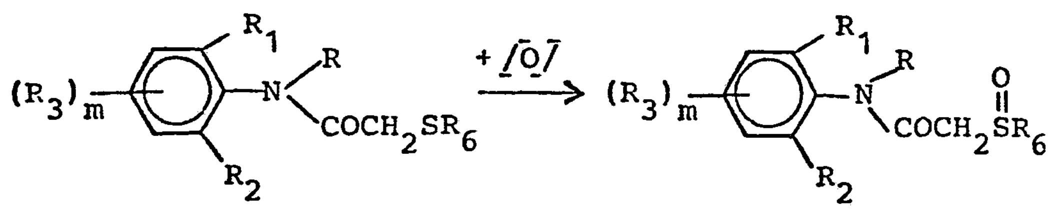

(VI) (I)

in der R, $R_1$, $R_2$, $R_3$, $R_6$ und m die Bedeutungen wie in Formel I haben.

Die Umsetzung nach dem Verfahren 1 wird bevorzugt in gegenüber den Reaktanten inerten, vorzugsweise polaren oder dipolaren aprotischen Lösungs- oder Verdünnungsmitteln durchgeführt. Als solche kommen z.B. in Frage: Alkohole wie Ethanol, Propanol, Butanol oder Glykol; dialkylierte Amide wie Dimethylformamid; Nitrile wie Acetonitril oder Propionitril; Ketone wie Methylethylketon oder Diisopropylketon; Ether wie Dioxan.

Die Reaktionstemperatur beträgt bevorzugt zwischen 50°C und 250°C, insbesondere zwischen 60 und 180°C. Als Literaturnachweise für die Umsetzung nach Verfahren 1 können z.B. Chem. Ber. 13, 1272 (1880), Chem. Ber. 63, 2847 (1930), Synthesis 1979, S. 733 genannt werden.

Die Umsetzungen nach den Verfahren 2 und 4 werden bevorzugt in inerten Lösungs- oder Verdünnungsmitteln durchgeführt. Man verwendet z.B. vorzugsweise Kohlenwasserstoffe wie Benzol, Toluol, Xylol, Petrolether, Cyclohexan; halogenierte Kohlenwasserstoffe wie Chlorbenzol, Tetrachlorkohlenstoff; Ether wie Dialkylether, Dioxan oder Tetrahydrofuran; Nitrile wie Acetonitril; dialkylierte

Amide wie Dimethylformamid oder Carbonsäureester wie Essigsäureethylester.

Die Reaktionstemperaturen sind nicht kritisch und liegen bevorzugt zwischen -20° und 160°C, insbesondere zwischen 0 und 140°C.

Die Umsetzungen nach den Verfahren 2 und 4 können mit oder ohne Anwesenheit von säurebindenden Mitteln durchgeführt werden. Als säurebindende Mittel kommen z.B. in Frage: Trialkylamine wie Triethylamin, Pyridin, anorganische Basen wie Metalloxide oder -hydroxide, z.B. Calziumoxid oder Natriumhydroxid, oder Carbonate wie Kaliumcarbonat in Frage. Wird die Umsetzung ohne säurebindende Mittel durchgeführt, so wird vorteilhaft bei erhöhter Temperatur gearbeitet und es muß für die Abführung des Halogenwasserstoffes aus dem Reaktionsgemisch Sorge getragen werden.

Die Herstellung der als Ausgangsmaterial verwendeten Anilinderivate der Formel IV kann z.B. erfolgen nach: J. Org. Chem. 30, 4101 (1965); DE-OS 22 12 268; US-PS 40 12 519; Tetrahedron 1967, 487 und 493.

Die Säurechloride der Formeln V und VII können aus Merkaptoessigsäuren oder deren Derivaten durch Oxidation und nachfolgende Überführung in das Säurechlorid durch Umsetzung mit Thionylchlorid hergestellt werden (vgl. Chem.Ber. 29, 1139 (1896)).

Die Oxidationen von Merkaptoacetaniliden der Formel VI nach den Verfahren 5 bzw. 3 zu Sulfoxiden bzw. Sulfonen können nach dem Fachmann geläufigen Methoden durchgeführt werden. Als Oxidationsmittel kommen z.B. in Frage: Wasserstoffperoxid /Chem.Ber. 41, 2836 (1908); Houben-Weyl Bd. 9, 4. Auflage, S. 228/, Persäuren wie Peressigsäure, Perbenzoesäure, 3-Chlorperbenzoesäure oder Phthalmonopersäure /Houben-Weyl, Bd. 9, 4. Auflage, S. 228;

J. prak. Chem. 131, 357 (1931) und 128, 171 (1930); Chem. Ber. 70, 379 (1937)], Chrom- oder Salpetersäure oder im Falle der Sulfoxide auch Brom oder Chlor [Synth. 1/1979 S. 39].

Die Umsetzungen nach den Verfahren 3 und 5 werden bevorzugt in Gegenwart von Lösungsmitteln durchgeführt. Als solche können verwendet werden:

Kohlenwasserstoffe wie z.B. Benzol, halogenierte Kohlenwasserstoffe wie Methylenchlorid, Chloroform, Tetrachlorchlorstoff, Dichlorethan oder Trichlorethylen, Ketone wie Aceton oder Methylethylketon, Carbonsäuren wie Eisessig oder Propionsäure, Anhydride wie Acetanhydrid, Alkohole wie Methanol, Äthanol und Wasser oder auch Gemische dieser Lösungsmittel untereinander.

Die Reaktionstemperaturen liegen bei dem Verfahren 3 zur Herstellung von Sulfonen zwischen -20 und 160°C, vorzugsweise zwischen 0 und 120°C, bei dem Verfahren 5 zur Herstellung von Sulfoxiden zwischen -40 und 100°C, vorzugsweise zwischen -40 und 60°C.

Die zur Umsetzung verwendeten Merkaptoacetanilide der Formel VI sind teilweise bekannt [vgl. DE-OS 25 15 091]. Ihre Herstellung kann z.B. durch Umsetzung von Anilinderivaten der Formel IV mit Merkaptoessigsäurechloriden oder durch Umsetzung von Halogenacetaniliden mit Merkaptanen bzw. deren Alkalisalzen erfolgen.

Wird die Herstellung der erfindungsgemäßen Verbindungen der Formel I nach einem der Verfahren 1 bis 5 in solchen Lösungsmitteln durchgeführt, in denen einer der Reaktanten nur wenig löslich ist, so kann eine Beschleunigung der Umsetzung durch die Zugabe von Katalysatoren, wie z.B. Kronenethern oder die als Phasentransferkatalysatoren bekannten quartären Ammonium-, bzw. Phosphoniumsalze erreicht werden.

Die Verbindungen der Formel I mit R = 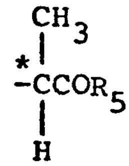 und

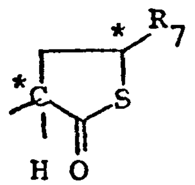

besitzen asymmetrische Kohlenstoffatome. Sie liegen daher im allgemeinen als racemische Gemische enantiomerer Formen vor. Die enantiomeren Formen weisen eine unterschiedliche biologische Aktivität auf. Eine gezielte Synthese des reinen D-Isomeren kann z.B. durch fraktionierte Kristallisation von Salzen der Anilinopropionsäure der allgemeinen Formel VIII mit optisch aktiven Basen

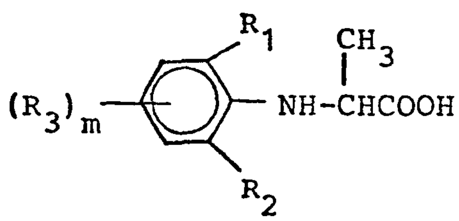

(VIII)

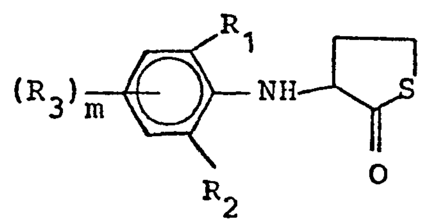

(VIII a)

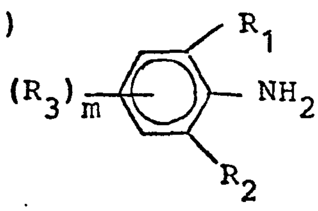

(IX)

wie Cinchonin oder α-Phenylethylamin oder durch fraktionierte Kristallisation von Anilinothiobutyrolactonen der allgemeinen Formel VIII a mit optisch aktiven Säuren, wie z.B. D- oder L-Weinsäure erfolgen. In den Formeln VIII und VIII a haben $R_1$, $R_2$, $R_3$ und m die Bedeutungen wie in Formel I.

Es ist aber auch eine gezielte Synthese, ausgehend von optisch aktiven Halogenpropionsäurederivaten, wie z.B. L-2-Chlorpropionsäuremethylester, optisch aktiven Milchsäureestertosylaten oder optisch aktiven Halogenbutyrothiolactonen und Anilinen der allgemeinen Formel IX, in der $R_1$, $R_2$, $R_3$ und m die Bedeutungen wie in Formel I haben, möglich. Diese Umsetzung erfolgt vorwiegend als SN2-Reaktion unter Inversion der Konfiguration.So entstehen aus 2,6-Dimethylanilin und L-2-Chlorpropionsäure bzw. L-2-Chlorpropionsäuremethylester bevorzugt D-2-(2,6-Dimethylanilino)-propionsäure bzw. deren Methylester.

Die erfindungsgemäßen Mittel enthalten die Wirkstoffe der Formel I im allgemeinen zu etwa 2-95 Gew.-%, vorzugsweise 5-90 Gew.-%. Sie können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenyl-sulfonate, und Dispergiermittel, z.B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:

Alkylarylsulfonsaure Calziumsalze wie Ca-dodecylbenzolsulfonat, oder nichtionische Emulgatoren wie Fettsäure-

polyglykolester, Alkylarylpolyglykolether, Fettalkohol-polyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylen-sorbitan-fettsäureester oder Polyoxethylen-sorbitester.

Stäubemittel kann man durch Vermahlen des Wirkstoffes mit fein verteilten, festen Stoffen, z.B. Talkum, natürlichen Tonen, wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen, wie Sand, Kaolinite, oder von granuliertem Inertmaterial. Auch können geeignete Wirkstoffe in der für die Herstellung von Düngemittelgranalien üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel-, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen pestiziden Wirkstoffen, wie z.B. Insektiziden, Akariziden, Herbiziden, Wachstumsregulatoren oder Fungiziden sind gegebenenfalls möglich. Insbesondere bei Mischungen mit Fungiziden können teilweise auch synergistische Wirkungssteigerungen erzielt werden.

Die Erfindung wird durch die folgenden Beispiele näher erläutert:

A. <u>Herstellungsbeispiele</u>

<u>Beispiel 1</u>

<u>Methyl-2-/N-(methylsulfonylacetyl)-N-(2,6-dimethylphenyl)-amino/-propanoat</u>

14,75 g (0,05 Mol) Methyl-2-/N-(methylthioacetyl)-N-(2,6-dimethylphenyl)-amino/-propanoat in 60 ml Methylenchlorid werden im Verlauf von 1 Stunde mit einer Suspension von 27,1 g (0,11 Mol) 3-Chlorperbenzoesäure (80 %ig) in 100 ml Methylenchlorid versetzt. Nach erfolgter Zugabe wird das Gemisch 2 Stunden auf 40°C erhitzt, die ausgeschiedene 3-Chlorbenzoesäure wird abfiltriert und das Filtrat mit 200 ml gesättigter Natriumbicarbonatlösung kräftig gerührt. Die Methylenchloridphase wird abgetrennt und über Natriumsulfat getrocknet. Danach wird das Methylenchlorid abdestilliert.

Der Rückstand wird aus Toluol/Cyclohexan umkristallisiert. Man erhält 14,1 g (= 86,3 % d. Theorie) Methyl-2-/N̄-(methylsulfonylacetyl)-N-(2,6-dimethylphenyl)-amino/-propanoat mit dem Schmelzpunkt 115 - 116°C. Formel:

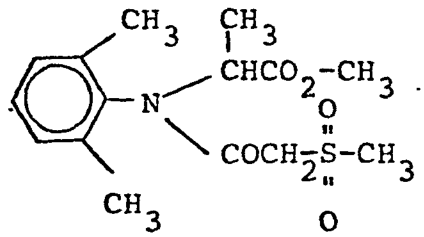

Beispiel 2

Methyl-2-/N̄-(ethylsulfonylacetyl)-N-(2,6-dimethylphenyl)-amino/-propanoat

28,3 g (0,1 Mol) Methyl-2-/N̄-(chloracetyl)-N-(2,6-dimethylphenyl)-amino/-propanoat, 1 g TEBA (= Triethylbenzylammoniumbromid) und 14,0 g (0,12 Mol) Natriumethylsulfinat werden in 100 ml wasserfreiem Dimethylformamid bei 120°C 40 Stunden erhitzt. Anschließend wird das Gemisch abfiltriert, das Dimethylformamid abdestilliert, der Rückstand in Wasser digeriert und mit Methylenchlorid extrahiert. Die Methylenchloridphase wird getrocknet und das Methylenchlorid abdestilliert. Der Rückstand wird aus Toluol/Cyclohexan umkristallisiert. Man erhält 23,5 g (= 69 % der Theorie) Methyl-2-/N̄-(ethylsulfonylacetyl)-N-(2,6-dimethylphenyl)-amino/-propanoat mit dem Schmelzpunkt 128 - 129°C. Formel:

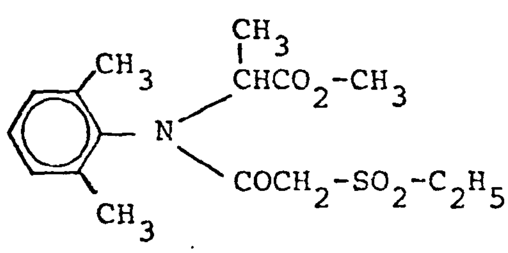

Beispiel 3

Methyl-2-/N-(n-propylsulfonylacetyl)-N-(2,6-dimethylphenyl)-amino7-propanoat

Zu einer Suspension von 20,7 g (0,1 Mol) Methyl-2-/N-2,6-dimethylphenyl)-amino7-propanoat und 13,8 g (0,1 Mol) Kaliumcarbonat in 100 ml trockenem Acetonitril gibt man bei Raumtemperatur 18,5 g (0,1 Mol) n-Propylsulfonylacetylchlorid. Nach 12-stündigem Rühren des Gemisches bei Raumtemperatur wird der Niederschlag von Kaliumhydrogencarbonat abfiltriert, das Filtrat auf 1 Liter Eiswasser gegeben und mit Methylenchlorid extrahiert. Die Methylenchloridphase wird getrocknet, anschließend das Methylenchlorid abdestilliert und der Rückstand aus Toluol umkristallisiert. Man erhält 22,4 g (= 63 % der Theorie) Methyl-2-/N-(n-propylsulfonylacetyl)-N-(2,6-dimethylphenyl)-amino7-propanoat mit dem Schmelzpunkt 124,5 - 125,5°C. Formel:

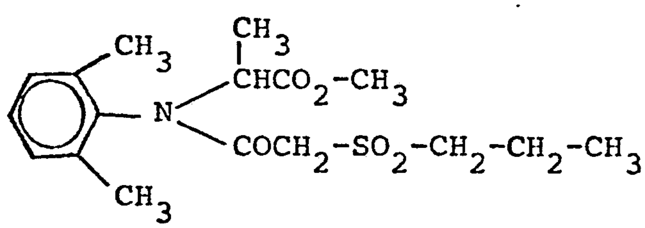

Beispiele 4 bis 112

Es werden analog zu den Beispielen 1 bis 3 die nachstehend in den Tabellen 1 und 2 aufgeführten Beispiele 4 bis 112 hergestellt. Es sind in der Tabelle 1 für die in den Beispielen 4 bis 95, und in der Tabelle 2 für die in den Beispielen 96 bis 112 hergestellten Verbindungen der Formel I die Reste $R_1$ bis $R_6$, n und m in zusammengefaßter Form wiedergegeben und jeweils in der letzten Tabellenspalte die ermittelten physikalisch-chemischen Kenndaten aufgeführt.

Tabelle 1

Formel I, n=1, m=0 oder 1:

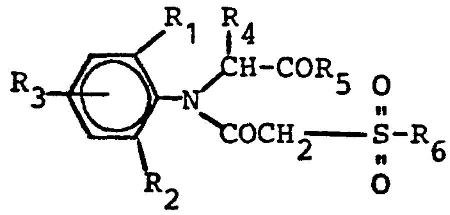

| Beispiel Nr. | $R_1$ | $R_2$ | $R_3$ | $R_4$ | $R_5$ | $R_6$ | physik.-chem. Kenndaten |
|---|---|---|---|---|---|---|---|
| 4 | $CH_3$ | $CH_3$ | H | $CH_3$ | $-OC_2H_5$ | $-CH_3$ | Fp. 129,5-130 °C |
| 5 | $CH_3$ | $CH_3$ | H | $CH_3$ | $-O-n-C_3H_7$ | $-CH_3$ | |
| 6 | $CH_3$ | $CH_3$ | H | $CH_3$ | $-O-i-C_3H_7$ | $-CH_3$ | |
| 7 | $CH_3$ | $CH_3$ | H | $CH_3$ | $-OCH_2CH(CH_3)_2$ | $-CH_3$ | |
| 8 | $CH_3$ | $CH_3$ | H | $CH_3$ | $-OCH_3$ | $i-C_3H_7$ | Fp. 127,5-128,5 °C |
| 9 | $CH_3$ | $CH_3$ | H | $CH_3$ | $-OCH_3$ | $t-C_4H_9$ | Fp. 105-107 °C |
| 10 | $CH_3$ | $CH_3$ | H | $CH_3$ | $-OCH_3$ | $s-C_4H_9$ | Fp. 121,5-122,5 °C |
| 11 | $CH_3$ | $CH_3$ | H | $CH_3$ | $-OCH_3$ | $i-C_4H_9$ | |
| 12 | $CH_3$ | $CH_3$ | H | $CH_3$ | $-OCH_3$ | $n-C_6H_{13}$ | |
| 13 | $CH_3$ | $CH_3$ | H | $CH_3$ | $-OCH_3$ | $-CH_2CH_2OCH_3$ | |
| 14 | $CH_3$ | $CH_3$ | H | $CH_3$ | $-OCH_3$ | $-CH_2CH_2OC_2H_5$ | $n_D^{23}$ = 1,5205 |
| 15 | $CH_3$ | $CH_3$ | H | $CH_3$ | $-OCH_3$ | $-CH_2CH_2CH_2Cl$ | |
| 16 | $CH_3$ | $CH_3$ | H | $CH_3$ | $-OCH_3$ | $-CH_2CH_2OH$ | Fp. 119-120 °C |
| 17 | $CH_3$ | $CH_3$ | H | $CH_3$ | $-OCH_3$ | $-CH_2CO_2CH_3$ | $n_D^{30}$ = 1,5270 |
| 18 | $CH_3$ | $CH_3$ | H | $CH_3$ | $-OCH_3$ | $-CH_2CH_2CO_2CH_3$ | |
| 19 | $CH_3$ | $CH_3$ | H | $CH_3$ | $-OCH_3$ | 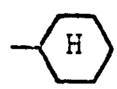 | |
| 20 | $CH_3$ | $CH_3$ | H | $CH_3$ | $-OCH_3$ | 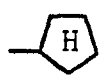 | |
| 21 | $CH_3$ | $CH_3$ | H | $CH_3$ | $-OCH_3$ | $-CH_2CH=CH_2$ | Fp. 93-93,5 °C |

(Tabelle 1, Fortsetzung)

| Bei-spiel Nr. | $R_1$ | $R_2$ | $R_3$ | $R_4$ | $R_5$ | $R_6$ | physik.-chem. Kenndaten |
|---|---|---|---|---|---|---|---|
| 22 | $CH_3$ | $CH_3$ | H | $CH_3$ | $-OCH_3$ | $-CH_2C{\equiv}CH$ | |
| 23 | $CH_3$ | $CH_3$ | H | $CH_3$ | $-OCH_3$ | $-CH_2$- 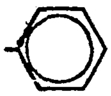 | Fp. 128 °C |
| 24 | $CH_3$ | $CH_3$ | H | $CH_3$ | $-OCH_3$ | 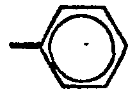 | Fp. 102 °C |
| 25 | $CH_3$ | $CH_3$ | H | $CH_3$ | $-OCH_3$ | 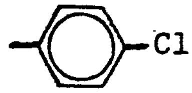 | Fp. 136 - 137,5°C |
| 26 | $CH_3$ | $CH_3$ | H | $CH_3$ | $-OCH_3$ | 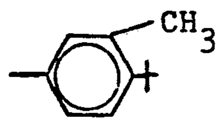 | Fp. 131,5 - 132,5°C |
| 27 | $CH_3$ | $CH_3$ | H | H | $-OCH_3$ | $-CH_3$ | Fp. 148 - 150 °C |
| 28 | $CH_3$ | $CH_3$ | H | H | $-OC_2H_5$ | $-CH_3$ | Fp. 85 - 87 °C |
| 29 | $CH_3$ | $CH_3$ | H | H | $-OCH_3$ | $-C_2H_5$ | |
| 30 | $CH_3$ | $CH_3$ | H | H | $-OCH_3$ | $n-C_3H_7$ | |
| 31 | $CH_3$ | $CH_3$ | H | H | $-OCH_3$ | $i-C_3H_7$ | |
| 32 | $CH_3$ | $CH_3$ | H | H | $-OCH_3$ | $s-C_4H_9$ | |
| 33 | $CH_3$ | $CH_3$ | H | H | $-OCH_3$ | $t-C_4H_9$ | |
| 34 | $CH_3$ | $CH_3$ | H | H | $-OCH_3$ | $-CH_2CH{=}CH_2$ | |
| 35 | $CH_3$ | $CH_3$ | H | H | $-OCH_3$ | $-CH_2CH_2OC_2H_5$ | |
| 36 | $CH_3$ | $C_2H_5$ | H | $CH_3$ | $-OCH_3$ | $-CH_3$ | Elementaranalyse:<br>% C H N<br>ber. 56,3 6,6 4,1<br>gef. 55,2 6,7 4,1 |
| 37 | $CH_3$ | $C_2H_5$ | H | $CH_3$ | $-OCH_3$ | $-C_2H_5$ | Fp. 93,5 - 95,5°C |
| 38 | $CH_3$ | $C_2H_5$ | H | $CH_3$ | $-OCH_3$ | $n-C_3H_7$ | |
| 39 | $CH_3$ | $C_2H_5$ | H | $CH_3$ | $-OCH_3$ | $i-C_3H_7$ | |
| 40 | $CH_3$ | $C_2H_5$ | H | $CH_3$ | $-OCH_3$ | $n-C_4H_9$ | |
| 41 | $CH_3$ | $C_2H_5$ | H | $CH_3$ | $-OCH_3$ | $i-C_4H_9$ | |
| 42 | $CH_3$ | $C_2H_5$ | H | $CH_3$ | $-OCH_3$ | $s-C_4H_9$ | |
| 43 | $CH_3$ | $C_2H_5$ | H | $CH_3$ | $-OCH_3$ | $t-C_4H_9$ | |

(Tabelle 1, Fortsetzung)

| Beispiel Nr. | $R_1$ | $R_2$ | $R_3$ | $R_4$ | $R_5$ | $R_6$ | physik.-chem. Kenndaten |
|---|---|---|---|---|---|---|---|
| 44 | $CH_3$ | $C_2H_5$ | H | $CH_3$ | $-OCH_3$ | $-CH_2-$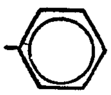 | |
| 45 | $CH_3$ | $C_2H_5$ | H | $CH_3$ | $-OCH_3$ | $-CH_2CH=CH_2$ | |
| 46 | $CH_3$ | $C_2H_5$ | H | $CH_3$ | $-OC_2H_5$ | $-CH_3$ | |
| 47 | $CH_3$ | $C_2H_5$ | H | $CH_3$ | $-OC_2H_5$ | $-C_2H_5$ | |
| 48 | $CH_3$ | $C_2H_5$ | H | $CH_3$ | $-OC_2H_5$ | $i-C_3H_7$ | |
| 49 | $CH_3$ | $C_2H_5$ | H | $CH_3$ | $-O-n-C_3H_7$ | $-CH_3$ | |
| 50 | $C_2H_5$ | $C_2H_5$ | H | $CH_3$ | $-OCH_3$ | $-CH_3$ | Fp. 93-96°C |
| 51 | $C_2H_5$ | $C_2H_5$ | H | $CH_3$ | $-OCH_3$ | $-C_2H_5$ | |
| 52 | $C_2H_5$ | $C_2H_5$ | H | $CH_3$ | $-OCH_3$ | $i-C_3H_7$ | |
| 53 | $C_2H_5$ | $C_2H_5$ | H | $CH_3$ | $-OC_2H_5$ | $-CH_3$ | $n_D^{27} = 1,5279$ |
| 54 | $C_2H_5$ | $C_2H_5$ | H | $CH_3$ | $-OC_2H_5$ | $-C_2H_5$ | $n_D^{26} = 1,5213$ |
| 55 | $C_2H_5$ | $i-C_3H_7$ | H | $CH_3$ | $-OCH_3$ | $-CH_3$ | |
| 56 | $C_2H_5$ | $i-C_3H_7$ | H | $CH_3$ | $-OCH_3$ | $-C_2H_5$ | |
| 57 | $i-C_3H_7$ | $i-C_3H_7$ | H | $CH_3$ | $-OCH_3$ | $-CH_3$ | |
| 58 | $i-C_3H_7$ | $i-C_3H_7$ | H | H | $-OCH_3$ | $-C_2H_5$ | |
| 59 | $CH_3$ | $C_2H_5$ | H | H | $-OCH_3$ | $-CH_3$ | Fp. 103-104°C |
| 60 | $CH_3$ | $C_2H_5$ | H | H | $-OCH_3$ | $-C_2H_5$ | |
| 61 | $CH_3$ | $C_2H_5$ | H | H | $-OCH_3$ | $i-C_3H_7$ | |
| 62 | $CH_3$ | $C_2H_5$ | H | H | $-OC_2H_5$ | $-CH_3$ | |
| 63 | $C_2H_5$ | $C_2H_5$ | H | H | $-OCH_3$ | $-CH_3$ | |
| 64 | $C_2H_5$ | $C_2H_5$ | H | H | $-OCH_3$ | $-C_2H_5$ | |
| 65 | $CH_3$ | $CH_3$ | H | $CH_3$ | $-OH$ | $-CH_3$ | Fp. 176°C |
| 66 | $CH_3$ | $CH_3$ | H | $CH_3$ | $-NH_2$ | $-CH_3$ | |
| 67 | $CH_3$ | $CH_3$ | H | $CH_3$ | $-NHCH_3$ | $-CH_3$ | |
| 68 | $CH_3$ | $CH_3$ | H | $CH_3$ | $-N(CH_3)_2$ | $-CH_3$ | Fp. 182°C |
| 69 | $CH_3$ | $CH_3$ | H | $CH_3$ | $-NHCH_3$ | $-C_2H_5$ | |
| 70 | $CH_3$ | $CH_3$ | H | $CH_3$ | $-N(CH_3)_2$ | $-C_2H_5$ | |
| 71 | $CH_3$ | $CH_3$ | H | $CH_3$ | $-S-C_2H_5$ | $-CH_3$ | |

(Tabelle 1, Fortsetzung)

| Bei-spiel Nr. | $R_1$ | $R_2$ | $R_3$ | $R_4$ | $R_5$ | $R_6$ | physik.-chem. Kenndaten |
|---|---|---|---|---|---|---|---|
| 72 | $CH_3$ | $CH_3$ | H | $CH_3$ | $-S-CH_3$ | $-CH_3$ | |
| 73 | $CH_3$ | H | H | $CH_3$ | $-OCH_3$ | $-CH_3$ | $n_D^{27} = 1,5344$ |
| 74 | $CH_3$ | H | H | $CH_3$ | $-OCH_3$ | $-C_2H_5$ | $n_D^{27} = 1,5300$ |
| 75 | $CH_3$ | H | H | $CH_3$ | $-OCH_3$ | $i-C_3H_7$ | |
| 76 | $CH_3$ | H | H | $CH_3$ | $-OCH_3$ | $n-C_3H_7$ | |
| 77 | $i-C_3H_7$ | H | H | $CH_3$ | $-OCH_3$ | $-CH_3$ | Fp. 128 -131°C |
| 78 | $i-C_3H_7$ | H | H | $CH_3$ | $-OCH_3$ | $-C_2H_5$ | |
| 79 | $i-C_3H_7$ | H | H | $CH_3$ | $-OCH_3$ | $i-C_3H_7$ | |
| 80 | $i-C_3H_7$ | H | H | H | $-OCH_3$ | $-CH_3$ | Fp. 105,5-106 °C |
| 81 | $CH_3$ | H | $3-CH_3$ | $CH_3$ | $-OCH_3$ | $-CH_3$ | $n_D^{22} = 1,5375$ |
| 82 | $CH_3$ | H | $3-CH_3$ | $CH_3$ | $-OCH_3$ | $-C_2H_5$ | |
| 83 | $CH_3$ | H | $4-CH_3$ | $CH_3$ | $-OCH_3$ | $-CH_3$ | |
| 84 | $CH_3$ | $CH_3$ | $3-CH_3$ | $CH_3$ | $-OCH_3$ | $-CH_3$ | |
| 85 | $CH_3$ | $CH_3$ | $3-CH_3$ | $CH_3$ | $-OCH_3$ | $-C_2H_5$ | |
| 86 | Cl | $CH_3$ | H | $CH_3$ | $-OCH_3$ | $-CH_3$ | Fp. 125 -127,5°C |
| 87 | Cl | $CH_3$ | H | $CH_3$ | $-OCH_3$ | $-C_2H_5$ | |
| 88 | Cl | $CH_3$ | H | $CH_3$ | $-OCH_3$ | $n-C_3H_7$ | |
| 89 | Cl | $CH_3$ | H | $CH_3$ | $-OCH_3$ | $i-C_3H_7$ | |
| 90 | $CH_3$ | $CH_3$ | H | $CH_3$ | $-OCH_3$ | $-CH_2-$ 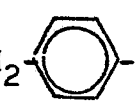 -Cl | |
| 91 | $CH_3$ | H | 5-Cl | $CH_3$ | $-OCH_3$ | $-CH_3$ | |
| 92 | Cl | H | 5-Cl | $CH_3$ | $-OCH_3$ | $-CH_3$ | |
| 93 | Cl | H | 3-Cl | $CH_3$ | $-OCH_3$ | $-CH_3$ | |
| 94 | Cl | Cl | H | $CH_3$ | $-OCH_3$ | $-CH_3$ | |
| 95 | Cl | Cl | H | $CH_3$ | $-OCH_3$ | $-C_2H_5$ | |

Tabelle 2

Formel I, n = 1, m = 0 oder 1:

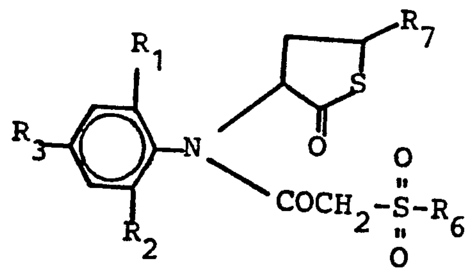

| Beispiel Nr. | $R_1$ | $R_2$ | $R_3$ | $R_6$ | $R_7$ | physik.-chem. Kenndaten |
|---|---|---|---|---|---|---|
| 96 | $CH_3$ | $CH_3$ | H | $-CH_3$ | H | |
| 97 | $CH_3$ | $CH_3$ | H | $-C_2H_5$ | H | |
| 98 | $CH_3$ | $CH_3$ | H | $n-C_3H_7$ | H | |
| 99 | $CH_3$ | $CH_3$ | H | $i-C_3H_7$ | H | |
| 100 | $CH_3$ | $CH_3$ | H | $-CH_2CH=CH_2$ | H | |
| 101 | $CH_3$ | $CH_3$ | H | $n-C_4H_9$ | H | |
| 102 | $CH_3$ | $CH_3$ | H | $i-C_4H_9$ | H | |
| 103 | $CH_3$ | $CH_3$ | H | $i-C_4H_9$ | H | |
| 104 | $CH_3$ | $CH_3$ | H | $t-C_4H_9$ | H | |
| 105 | $CH_3$ | $CH_3$ | H | 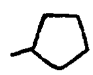 | H | |
| 106 | $CH_3$ | $CH_3$ | H | $-CH_2CH_2OC_2H_5$ | H | |
| 107 | $CH_3$ | $C_2H_5$ | H | $-CH_3$ | H | |
| 108 | $C_2H_5$ | $C_2H_5$ | H | $-CH_3$ | H | |
| 109 | $CH_3$ | $CH_3$ | $3-CH_3$ | $-CH_3$ | H | |
| 110 | Cl | $CH_3$ | H | $-CH_3$ | H | |
| 111 | $CH_3$ | $CH_3$ | H | $-CH_3$ | $-CH_3$ | |
| 112 | $CH_3$ | $CH_3$ | H | $-CH_2-$ 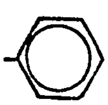 | H | |

Beispiel 113

Methyl-2-/N-(n-propylsulfinylacetyl)-N-(2,6-dimethylphenyl)-amino/-propanoat

Einer Lösung von 16,15 g (0,05 Mol) Methyl-2-/N-(n-propylthioacetyl)-N-(2,6-dimethylphenyl)-amino/-propanoat in 60 ml Methylenchlorid wird bei -20 bis -30°C innerhalb von 2 Stunden unter Rühren eine Lösung von 10,15 g (0,05 Mol) 85 %ige 3-Chlorperbenzoesäure in 100 ml Methylenchlorid zudosiert. Nach 2-stündigem weiterem Rühren bei Raumtemperatur wird das Gemisch abfiltriert, die Methylenchloridphase intensiv mit 200 ml gesättigter, wässriger Natriumbicarbonatlösung gewaschen und über Natriumsulfat getrocknet. Anschließend wird das Methylenchlorid abdestilliert und der Rückstand aus Toluol/Cyclohexan umkristallisiert. Man erhält 14,3 g (= 84,5 % der Theorie) Methyl-2-/N-(n-propylsulfinylacetyl)-N-(2,6-dimethylphenyl)-amino/-propanoat mit dem Schmelzpunkt 93 - 95°C. Formel:

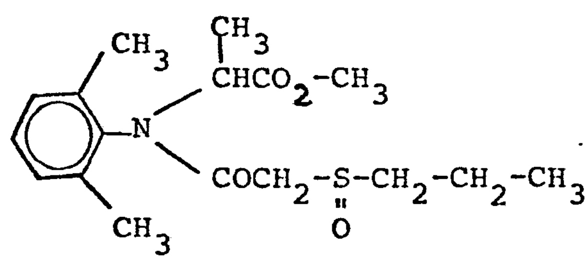

Beispiel 114

Methyl-2-/N-(phenylsulfinylacetyl)-N-(2,6-dimethylphenyl)-amino/-propanoat

17,86 g (0,05 Mol) Methyl-2-/N-(phenylthioacetyl)-N-(2,6-dimethylphenyl)-amino/-propanoat, gelöst in 100 ml Methylenchlorid, und 100 ml 10 %ige wässrige Kaliumhydrogencarbonatlösung werden in einem 500 ml Vierhalskolben vorgelegt. Unter guter Rührung gibt man zu dem Gemisch bei Raumtemperatur 8 g Brom und rührt 2 Stunden bei Raumtemperatur nach. Anschließend wird die Methylenchloridphase

abgetrennt und über Natriumsulfat getrocknet. Danach wird das Methylenchlorid abdestilliert und der Rückstand aus Toluol umkristallisiert. Man erhält 15,2 g (= 81 % der Theorie) Methyl-2-/N-(phenylsulfinylacetyl)-N-(2,6-dimethylphenyl)-amino7-propanoat mit dem Schmelzpunkt 116 - 118°C. Formel:

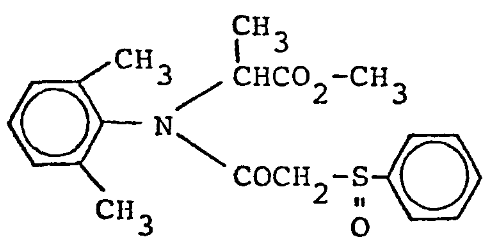

Beispiele 115 - 225

Es werden analog zu den Beispielen 113 und 114 die nachstehend in den Tabellen 3 und 4 aufgeführten Beispiele 115 bis 225 hergestellt. Es sind in der Tabelle 3 für die in den Beispielen 115 bis 208, und in der Tabelle 4 für die in den Beispielen 209 bis 225 hergestellten Verbindungen der Formel I die Reste $R_1$ bis $R_6$, n und m in zusammengefaßter Form wiedergegeben und jeweils in der letzten Tabellenspalte die ermittelten physikalisch-chemischen Kenndaten aufgeführt.

In der Tabelle 5 ist zusätzlich eine Auswahl von weiteren 10 Beispielen mit optisch aktiven Verbindungen der Formel I der D-Reihe bzw. der L-Reihe aufgeführt. Diese Produkte werden von den Beispielen mit den entsprechenden racemischen Produkten durch den Zusatz "AD" bzw. "AL" zur Beispiel-Nr. unterschieden. In der Tabelle 5 werden diese optisch aktiven Verbindungen neben den entsprechenden Substituenten in Formel I außerdem durch ihre spezifische Drehung $[\alpha]_D^{t°C}$ charakterisiert. Ihre Herstellung erfolgt in Analogie zu den entsprechenden racemischen Produkten.

Tabelle 3

Formel I, n=0, m=0 oder 1:

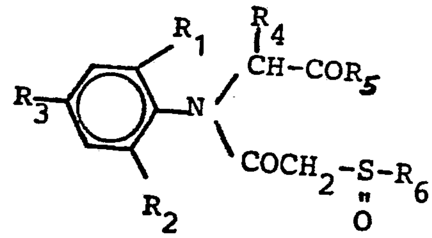

| Beispiel Nr. | $R_1$ | $R_2$ | $R_3$ | $R_4$ | $R_5$ | $R_6$ | physik.-chem. Kenndaten |
|---|---|---|---|---|---|---|---|
| 115 | $CH_3$ | $CH_3$ | H | $CH_3$ | $-OCH_3$ | $-CH_3$ | Fp. 78°C |
| 116 | $CH_3$ | $CH_3$ | H | $CH_3$ | $-OC_2H_5$ | $-CH_3$ | $n_D^{25}$ = 1,5403 |
| 117 | $CH_3$ | $CH_3$ | H | $CH_3$ | $-O-n-C_3H_7$ | $-CH_3$ | |
| 118 | $CH_3$ | $CH_3$ | H | $CH_3$ | $-O-i-C_3H_7$ | $-CH_3$ | |
| 119 | $CH_3$ | $CH_3$ | H | $CH_3$ | $-OCH_2CH(CH_3)_2$ | $-CH_3$ | |
| 120 | $CH_3$ | $CH_3$ | H | $CH_3$ | $-OCH_3$ | $-C_2H_5$ | Fp. 124 - 127 °C |
| 121 | $CH_3$ | $CH_3$ | H | $CH_3$ | $-OCH_3$ | $i-C_3H_7$ | $n_D^{27}$ = 1,5363 |
| 122 | $CH_3$ | $CH_3$ | H | $CH_3$ | $-OCH_3$ | $t-C_4H_9$ | Fp. 103 - 106 °C |
| 123 | $CH_3$ | $CH_3$ | H | $CH_3$ | $-OCH_3$ | $s-C_4H_9$ | $n_D^{27}$ = 1,5339 |
| 124 | $CH_3$ | $CH_3$ | H | $CH_3$ | $-OCH_3$ | $i-C_4H_9$ | |
| 125 | $CH_3$ | $CH_3$ | H | $CH_3$ | $-OCH_3$ | $n-C_6H_{13}$ | |
| 126 | $CH_3$ | $CH_3$ | H | $CH_3$ | $-OCH_3$ | $-CH_2CH_2OCH_3$ | |
| 127 | $CH_3$ | $CH_3$ | H | $CH_3$ | $-OCH_3$ | $-CH_2CH_2OC_2H_5$ | |
| 128 | $CH_3$ | $CH_3$ | H | $CH_3$ | $-OCH_3$ | $-CH_2CH_2CH_2Cl$ | |
| 129 | $CH_3$ | $CH_3$ | H | $CH_3$ | $-OCH_3$ | $-CH_2CH_2OH$ | Fp. 123 - 124,5°C |
| 130 | $CH_3$ | $CH_3$ | H | $CH_3$ | $-OCH_3$ | $-CH_2CO_2CH_3$ | Fp. 131 - 133 °C |
| 131 | $CH_3$ | $CH_3$ | H | $CH_3$ | $-OCH_3$ | $-CH_2CH_2CO_2CH_3$ | |
| 132 | $CH_3$ | $CH_3$ | H | $CH_3$ | $-OCH_3$ | 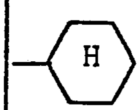 | |
| 133 | $CH_3$ | $CH_3$ | H | $CH_3$ | $-OCH_3$ | 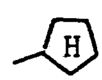 | |
| 134 | $CH_3$ | $CH_3$ | H | $CH_3$ | $-OCH_3$ | $-CH_2CH=CH_2$ | $n_D^{23}$ = 1,5430 |

(Tabelle 3, Fortsetzung)

| Beispiel Nr. | $R_1$ | $R_2$ | $R_3$ | $R_4$ | $R_5$ | $R_6$ | physik.-chem. Kenndaten |
|---|---|---|---|---|---|---|---|
| 135 | $CH_3$ | $CH_3$ | H | $CH_3$ | $-OCH_3$ | $-CH_2C{\equiv}CH$ | |
| 136 | $CH_3$ | $CH_3$ | H | $CH_3$ | $-OCH_3$ | $-CH_2$- 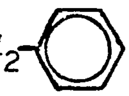 | $n_D^{23} = 1{,}5612$ |
| 137 | $CH_3$ | $CH_3$ | H | $CH_3$ | $-OCH_3$ | 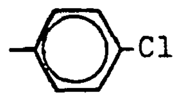 | |
| 138 | $CH_3$ | $CH_3$ | H | $CH_3$ | $-OCH_3$ | 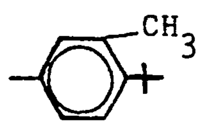 | |
| 139 | $CH_3$ | $CH_3$ | H | H | $-OCH_3$ | $-CH_3$ | Fp. 106 - 108 °C |
| 140 | $CH_3$ | $CH_3$ | H | H | $-OC_2H_5$ | $-CH_3$ | Fp. 74 - 75 °C |
| 141 | $CH_3$ | $CH_3$ | H | H | $-OCH_3$ | $-C_2H_5$ | |
| 142 | $CH_3$ | $CH_3$ | H | H | $-OCH_3$ | $n-C_3H_7$ | |
| 143 | $CH_3$ | $CH_3$ | H | H | $-OCH_3$ | $i-C_3H_7$ | |
| 144 | $CH_3$ | $CH_3$ | H | H | $-OCH_3$ | $s-C_4H_9$ | |
| 145 | $CH_3$ | $CH_3$ | H | H | $-OCH_3$ | $t-C_4H_9$ | |
| 146 | $CH_3$ | $CH_3$ | H | H | $-OCH_3$ | $-CH_2CH{=}CH_2$ | |
| 147 | $CH_3$ | $CH_3$ | H | H | $-OCH_3$ | $-CH_2CH_2OC_2H_5$ | |
| 148 | $CH_3$ | $C_2H_5$ | H | $CH_3$ | $-OCH_3$ | $-CH_3$ | $n_D^{22} = 1{,}5425$ |
| 149 | $CH_3$ | $C_2H_5$ | H | $CH_3$ | $-OCH_3$ | $-C_2H_5$ | $n_D^{22} = 1{,}5393$ |
| 150 | $CH_3$ | $C_2H_5$ | H | $CH_3$ | $-OCH_3$ | $n-C_3H_7$ | |
| 151 | $CH_3$ | $C_2H_5$ | H | $CH_3$ | $-OCH_3$ | $i-C_3H_7$ | |
| 152 | $CH_3$ | $C_2H_5$ | H | $CH_3$ | $-OCH_3$ | $n-C_4H_9$ | |
| 153 | $CH_3$ | $C_2H_5$ | H | $CH_3$ | $-OCH_3$ | $i-C_4H_9$ | |
| 154 | $CH_3$ | $C_2H_5$ | H | $CH_3$ | $-OCH_3$ | $s-C_4H_9$ | |
| 155 | $CH_3$ | $C_2H_5$ | H | $CH_3$ | $-OCH_3$ | $t-C_4H_9$ | |

(Tabelle 3, Fortsetzung)

| Bei-spiel Nr. | $R_1$ | $R_2$ | $R_3$ | $R_4$ | $R_5$ | $R_6$ | physik.-chem. Kenndaten |
|---|---|---|---|---|---|---|---|
| 156 | $CH_3$ | $C_2H_5$ | H | $CH_3$ | $-OCH_3$ | $-CH_2-$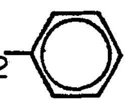 | |
| 157 | $CH_3$ | $C_2H_5$ | H | $CH_3$ | $-OCH_3$ | $-CH_2CH=CH_2$ | |
| 158 | $CH_3$ | $C_2H_5$ | H | $CH_3$ | $-OC_2H_5$ | $-CH_3$ | |
| 159 | $CH_3$ | $C_2H_5$ | H | $CH_3$ | $-OC_2H_5$ | $-C_2H_5$ | |
| 160 | $CH_3$ | $C_2H_5$ | H | $CH_3$ | $-OC_2H_5$ | $i-C_3H_7$ | |
| 161 | $CH_3$ | $C_2H_5$ | H | $CH_3$ | $-O-n-C_3H_7$ | $-CH_3$ | |
| 162 | $C_2H_5$ | $C_2H_5$ | H | $CH_3$ | $-OCH_3$ | $-CH_3$ | |
| 163 | $C_2H_5$ | $C_2H_5$ | H | $CH_3$ | $-OCH_3$ | $-C_2H_5$ | |
| 164 | $C_2H_5$ | $C_2H_5$ | H | $CH_3$ | $-OCH_3$ | $i-C_3H_7$ | |
| 165 | $C_2H_5$ | $C_2H_5$ | H | $CH_3$ | $-OC_2H_5$ | $-CH_3$ | $n_D^{27} = 1,5356$ |
| 166 | $C_2H_5$ | $C_2H_5$ | H | $CH_3$ | $-OC_2H_5$ | $-C_2H_5$ | $n_D^{22} = 1,5320$ |
| 167 | $C_2H_5$ | $i-C_3H_7$ | H | $CH_3$ | $-OCH_3$ | $-CH_3$ | |
| 168 | $C_2H_5$ | $i-C_3H_7$ | H | $CH_3$ | $-OCH_3$ | $-C_2H_5$ | |
| 169 | $i-C_3H_7$ | $i-C_3H_7$ | H | $CH_3$ | $-OCH_3$ | $-CH_3$ | |
| 170 | $i-C_3H_7$ | $i-C_3H_7$ | H | $CH_3$ | $-OCH_3$ | $-C_2H_5$ | |
| 171 | $CH_3$ | $C_2H_5$ | H | H | $-OCH_3$ | $-CH_3$ | Fp. 85 - 88°C |
| 172 | $CH_3$ | $C_2H_5$ | H | H | $-OCH_3$ | $-C_2H_5$ | |
| 173 | $CH_3$ | $C_2H_5$ | H | H | $-OCH_3$ | $i-C_3H_7$ | |
| 174 | $CH_3$ | $C_2H_5$ | H | H | $-OC_2H_5$ | $-CH_3$ | |
| 175 | $C_2H_5$ | $C_2H_5$ | H | H | $-OCH_3$ | $-CH_3$ | |
| 176 | $C_2H_5$ | $C_2H_5$ | H | H | $-OCH_3$ | $-C_2H_5$ | |
| 177 | $CH_3$ | $CH_3$ | H | $CH_3$ | $-OH$ | $-CH_3$ | |
| 178 | $CH_3$ | $CH_3$ | H | $CH_3$ | $-NH_2$ | $-CH_3$ | |
| 179 | $CH_3$ | $CH_3$ | H | $CH_3$ | $-NHCH_3$ | $-CH_3$ | |
| 180 | $CH_3$ | $CH_3$ | H | $CH_3$ | $-N(CH_3)_2$ | $-CH_3$ | |
| 181 | $CH_3$ | $CH_3$ | H | $CH_3$ | $-NHCH_3$ | $-C_2H_5$ | |
| 182 | $CH_3$ | $CH_3$ | H | $CH_3$ | $-N(CH_3)_2$ | $-C_2H_5$ | |

(Tabelle 3, Fortsetzung)

| Bei-spiel Nr. | $R_1$ | $R_2$ | $R_3$ | $R_4$ | $R_5$ | $R_6$ | physik.-chem. Kenndaten |
|---|---|---|---|---|---|---|---|
| 183 | $CH_3$ | $CH_3$ | H | $CH_3$ | $-S-C_2H_5$ | $-CH_3$ | |
| 184 | $CH_3$ | $CH_3$ | H | $CH_3$ | $-S-CH_3$ | $-CH_3$ | |
| 185 | $CH_3$ | H | H | $CH_3$ | $-OCH_3$ | $-CH_3$ | $n_D^{22}$= 1,5448 |
| 186 | $CH_3$ | H | H | $CH_3$ | $-OCH_3$ | $-C_2H_5$ | $n_D^{22}$= 1,5398 |
| 187 | $CH_3$ | H | H | $CH_3$ | $-OCH_3$ | $i-C_3H_7$ | |
| 188 | $CH_3$ | H | H | $CH_3$ | $-OCH_3$ | $n-C_3H_7$ | |
| 189 | - | - | - | - | - | - | |
| 190 | $i-C_3H_7$ | H | H | $CH_3$ | $-OCH_3$ | $-CH_3$ | Fp. 140°C |
| 191 | $i-C_3H_7$ | H | H | $CH_3$ | $-OCH_3$ | $-C_2H_5$ | |
| 192 | $i-C_3H_7$ | H | H | $CH_3$ | $-OCH_3$ | $i-C_3H_7$ | |
| 193 | $i-C_3H_7$ | H | H | H | $-OCH_3$ | $-CH_3$ | Fp. 134-135°C |
| 194 | $CH_3$ | H | $3-CH_3$ | $CH_3$ | $-OCH_3$ | $-CH_3$ | $n_D^{30}$= 1,5390 |
| 195 | $CH_3$ | H | $3-CH_3$ | $CH_3$ | $-OCH_3$ | $-C_2H_5$ | |
| 196 | $CH_3$ | H | $4-CH_3$ | $CH_3$ | $-OCH_3$ | $-CH_3$ | |
| 197 | $CH_3$ | $CH_3$ | $3-CH_3$ | $CH_3$ | $-OCH_3$ | $-CH_3$ | |
| 198 | $CH_3$ | $CH_3$ | $3-CH_3$ | $CH_3$ | $-OCH_3$ | $-C_2H_5$ | |
| 199 | Cl | $CH_3$ | H | $CH_3$ | $-OCH_3$ | $-CH_3$ | |
| 200 | Cl | $CH_3$ | H | $CH_3$ | $-OCH_3$ | $-C_2H_5$ | |
| 201 | Cl | $CH_3$ | H | $CH_3$ | $-OCH_3$ | $n-C_3H_7$ | |
| 202 | Cl | $CH_3$ | H | $CH_3$ | $-OCH_3$ | $i-C_3H_7$ | |
| 203 | $CH_3$ | $CH_3$ | H | $CH_3$ | $-OCH_3$ | $-CH_2$- 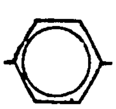 -Cl | |
| 204 | $CH_3$ | H | 5-Cl | $CH_3$ | $-OCH_3$ | $-CH_3$ | |
| 205 | Cl | H | 5-Cl | $CH_3$ | $-OCH_3$ | $-CH_3$ | |
| 206 | Cl | H | 3-Cl | $CH_3$ | $-OCH_3$ | $-CH_3$ | |
| 207 | Cl | Cl | H | $CH_3$ | $-OCH_3$ | $-CH_3$ | |
| 208 | Cl | Cl | H | $CH_3$ | $-OCH_3$ | $-C_2H_5$ | |

Tabelle 4

Formel I, n = 0, m = 0 oder 1:

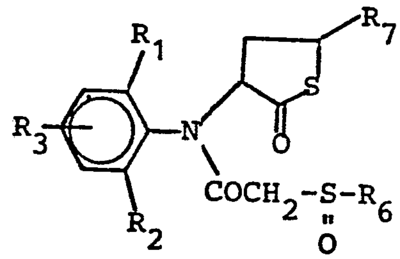

| Bei-spiel Nr. | $R_1$ | $R_2$ | $R_3$ | $R_6$ | $R_7$ | physik.-chem. Kenndaten |
|---|---|---|---|---|---|---|
| 209 | $CH_3$ | $CH_3$ | H | $-CH_3$ | H | |
| 210 | $CH_3$ | $CH_3$ | H | $-C_2H_5$ | H | |
| 211 | $CH_3$ | $CH_3$ | H | $n-C_3H_7$ | H | |
| 212 | $CH_3$ | $CH_3$ | H | $i-C_3H_7$ | H | |
| 213 | $CH_3$ | $CH_3$ | H | $-CH_2CH=CH_2$ | H | |
| 214 | $CH_3$ | $CH_3$ | H | $n-C_4H_9$ | H | |
| 215 | $CH_3$ | $CH_3$ | H | $i-C_4H_9$ | H | |
| 216 | $CH_3$ | $CH_3$ | H | $s-C_4H_9$ | H | |
| 217 | $CH_3$ | $CH_3$ | H | $t-C_4H_9$ | H | |
| 218 | $CH_3$ | $CH_3$ | H | 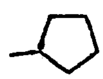 | H | |
| 219 | $CH_3$ | $CH_3$ | H | $-CH_2CH_2OC_2H_5$ | H | |
| 220 | $CH_3$ | $C_2H_5$ | H | $-CH_3$ | H | |
| 221 | $C_2H_5$ | $C_2H_5$ | H | $-CH_3$ | H | |
| 222 | $CH_3$ | $CH_3$ | $3-CH_3$ | $-CH_3$ | H | |
| 223 | Cl | $CH_3$ | H | $-CH_3$ | H | |
| 224 | $CH_3$ | $CH_3$ | H | $-CH_3$ | $-CH_3$ | |
| 225 | $CH_3$ | $CH_3$ | H | $-CH_2-$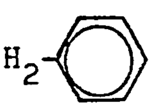 | $-CH_3$ | |

Tabelle 5

Formel I, $R_3$= H, m= O, R= $-\overset{R_4}{\overset{|}{C}}HCOR_5$, $R_4$= $-CH_3$:

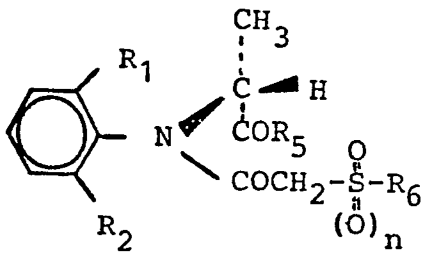

D-Form

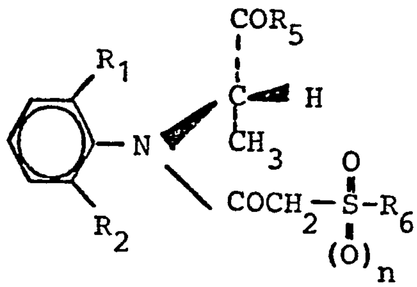

L-Form

| Beispiel Nr. | $R_1$ | $R_2$ | $R_5$ | $R_6$ | n | $[\alpha]_D^{t°C}$ spezif. Drehung |
|---|---|---|---|---|---|---|
| 1 AD | $-CH_3$ | $-CH_3$ | $-OCH_3$ | $-CH_3$ | 1 | $[\alpha]_D^{22°C}$= -36,86° |
| 1 AL | $-CH_3$ | $-CH_3$ | $-OCH_3$ | $-CH_3$ | 1 | $[\alpha]_D^{22°C}$= + 38,5° |
| 2 AD | $-CH_3$ | $-CH_3$ | $-OCH_3$ | $-C_2H_5$ | 1 | |
| 3 AD | $-CH_3$ | $-CH_3$ | $-OCH_3$ | $n-C_3H_7$ | 1 | |
| 8 AD | $-CH_3$ | $-CH_3$ | $-OCH_3$ | $i-C_3H_7$ | 1 | |
| 72 AD | $-CH_3$ | $-CH_3$ | $-SCH_3$ | $-CH_3$ | 1 | |
| 113 AD | $-CH_3$ | $-CH_3$ | $-OCH_3$ | $n-C_3H_7$ | 0 | |
| 115 AD | $-CH_3$ | $-CH_3$ | $-OCH_3$ | $-CH_3$ | 0 | $[\alpha]_D^{20°C}$= - 40,2°C |
| 120 AD | $-CH_3$ | $-CH_3$ | $-OCH_3$ | $-C_2H_5$ | 0 | |
| 121 AD | $-CH_3$ | $-CH_3$ | $-OCH_3$ | $i-C_3H_7$ | 0 | |

## B. Formulierungsbeispiele

### Beispiel A

Ein Stäubemittel wird erhalten, indem man 10 Gewichtsteile Wirkstoff und 90 Gewichtsteile Talkum als Inertstoff mischt und in einer Schlagmühle zerkleinert.

### Beispiel B

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

### Beispiel C

Ein in Wasser leicht dispergierbares Dispersionskonzentrat wird erhalten, indem man 20 Gewichtsteile Wirkstoff mit 6 Gewichtsteilen Nonylphenolpolyglykolether (10 AeO)*), 3 Gewichtsteilen Isotridecanolpolyglykolether (8 AeO)*) und 71 Gewichtsteilen paraffinischem Mineralöl (Siedebereich z.B. ca. 255 bis über 377°C) mischt und in einer Reibkugelmühle auf eine Feinheit von unter 5 Mikron vermahlt.

*) = Anzahl Ethylenoxydeinheiten im Polyglykoletherrest

### Beispiel D

Ein emulgierbares Konzentrat wird erhalten aus 15 Gewichtsteilen Wirkstoff, 75 Gewichtsteilen Cyclohexanon als Lösungsmittel und 10 Gewichtsteilen Nonylphenolpolyglykolether (10 AeO) als Emulgator.

Beispiel E

Zur Herstellung eines 5 % Wirkstoff enthaltenden Granulates werden die folgenden Komponenten verwendet: 5 Gew.-Teile Wirkstoff, 0,25 Gew.-Teile Epichlorhydrin, 0,25 Gew.-Teile Cetylpolyglykolether, 3,5 Gew.-Teile Polyethylenglykol, 91 Gew.-Teile Kaolin. Es werden der Wirkstoff mit dem Epichlorhydrin vermischt und in 6 Gew.-Teilen Aceton gelöst, dann das Polyethylenglykol und der Cetylether zugesetzt. Die resultierende Lösung wird auf Kaolin aufgesprüht und anschließend das Aceton unter Vakuum verdampft.

C. Biologische Beispiele

Beispiel I

Wirkung der Verbindungen der Formel I gegen Pythium ultimum an Erbsen

a) Wirkung nach Bodenapplikation

Der Pilz wird in einer Sandkultur angezogen und mit Erde vermischt. Die so infizierte Erde wird in Anzuchttöpfe abgefüllt und mit Erbsensaatgut besät. Nach der Aussaat werden die zu prüfenden fungiziden Verbindungen als wässrige Suspensionen jeweils auf die Oberfläche des Kulturbodens gegossen. Dabei werden Wirkstoffkonzentrationen von 200 bis 12 ppm (bezogen auf das Bodenvolumen) angewandt. Die Töpfe werden 2-3 Wochen in ein Gewächshaus mit 20 bis 22°C aufgestellt und der Kulturboden gleichmäßig leicht feuchtgehalten. Bei der Auswertung auf Pythium-Befall werden der Auflauf der Erbsenpflanzen sowie der Anteil gesunder und kranker Pflanzen im Vergleich zu den entsprechenden Kontrollen ermittelt und entsprechend der Wirkungsgrad errechnet.

Die beanspruchten Verbindungen der Formel I führen bei einer Aufwandmenge von 200 ppm zu einer weitgehenden

Kontrolle des Pythium ultimum-Befalls (Wirkungsgrad durchschnittlich > 80 %). Eine Reihe von Verbindungen der Formel I ist bei noch wesentlich geringerer Wirkstoffkonzentration ausgezeichnet wirksam, wie die in der nachfolgenden Tabelle I a) zusammengefaßten Versuchsergebnisse zeigen.

Tabelle I a

Wirkung gegen Pythium ultimum nach Behandlung von beimpfter Erde und anschließender Aussaat von gesundem Saatgut.

1. Tag: Beimpfung des Bodens mit Pythium ultimum und Behandlung mit fungizider Verbindung, Aussaat

14. Tag: Auswertung.

| Verbindung gemäß Beispiel Nr. | Wirkungsgrad in % bei ppm Wirkstoff im Boden | | |
|---|---|---|---|
| | 200 (ppm) | 100 (ppm) | 50 (ppm) |
| 1 | 100 | 100 | 100 |
| 9 | 100 | 100 | 100 |
| 113 | 100 | 100 | 100 |
| 3 | 100 | 100 | 100 |
| 2 | 100 | 100 | 100 |
| 120 | 100 | 100 | 100 |
| 21 | 100 | 100 | 100 |
| 115 | 100 | 100 | 100 |
| 8 | 100 | 100 | 100 |
| 134 | 100 | 100 | 100 |
| 121 | 100 | 100 | 100 |
| 129 | 100 | 100 | 100 |
| 148 | 100 | 100 | 100 |
| 86 | 100 | 100 | 100 |
| 130 | 100 | 85 | |

| Verbindung gemäß Beispiel Nr. | Wirkungsgrad in % bei ppm Wirkstoff im Boden 200 (ppm) | 100 (ppm) | 50 (ppm) |
|---|---|---|---|
| 14 | 100 | 100 | 100 |
| 16 | 100 | 100 | 100 |
| 17 | 100 | 100 | 100 |
| Vergleichsmittel A*) | 65 | 40 | 0 |
| Kontrolle: | | | |
| unbehandelte, beimpfte Erde | 0 ( = 100 % Auflaufschäden) | | |
| unbehandelte, nicht beimpfte Erde | 100 % Auflauf | | |

*)Vergleichsmittel A: Methyl-2-/N-(methylthioacetyl)-N-(2,6-dimethylphenyl)-amino/-propanoat (vgl. DE-OS 25 15 091).

b) Wirkung nach Beizapplikation

Der Pilz wird wie in dem Beispiel I a) beschrieben, angezogen und mit der Versuchserde vermischt. Diese wird in Anzuchttöpfe abgefüllt und mit Erbsensamen besät, die zuvor mit den zu prüfenden und als Beizmittel formulierten Versuchspräparaten gebeizt worden waren. Dabei werden Wirkstoffkonzentrationen von 1000 - 250 ppm (bezogen auf das Saatgutgewicht) angewandt. Die Töpfe werden 2-3 Wochen im Gewächshaus bei 20-22°C aufgestellt und die Erde gleichmäßig leicht feuchtgehalten. Bei der Auswertung wird wie in dem Beispiel I a) beschrieben, verfahren.

Die Verbindungen der Formel I zeigen bei 1000 ppm einen durchschnittlichen Wirkungsgrad von > 80 %. Eine Reihe von Verbindungen der Formel I ergeben bei noch wesentlich geringerer Konzentration eine vollständige Befallskontrolle, wie die in der nachfolgenden Tabelle I b) zusammengefaßten Versuchsergebnisse zeigen.

Tabelle I b

Wirkung gegen Pythium ultimum nach Behandlung (Beizung) des Erbsensaatgutes und anschließender Aussaat in beimpfte Erde.

1. Tag: Beimpfung der Versuchserde und Aussaat der gebeizten Erbsensamen sowie der ungebeizten Kontrollversuche.

14. Tag: Auswertung.

| Verbindung gemäß Beispiel Nr. | Wirkungsgrad in % bei ppm Wirkstoff auf dem Saatgut 1000 (ppm) | 500 (ppm) |
|---|---|---|
| 1 | 100 | 100 |
| 9 | 100 | 100 |
| 2 | 100 | 100 |
| 3 | 100 | 100 |
| 115 | 100 | 100 |
| 121 | 100 | 100 |
| 134 | 100 | 100 |
| 8 | 100 | 100 |
| Kontrolle: | | |
| ungebeiztes Saatgut, beimpfte Erde | 0 ( = 100 % Auflaufschäden) | |
| ungebeiztes Saatgut, gedämpfte Erde | 100 % Auflauf | |

Beispiel II

Präventive Wirkung der Verbindungen der Formel I gegen Phytophthora capsici (Bodenapplikation)

Phytophthora capsici wird in Sandkulturen angezogen und mit Versuchserde vermischt. Der so infizierte Boden wird in Anzuchttöpfe abgefüllt und mit Paprika (Capsicum annuum) besät. Nach der Aussaat werden die beanspruchten Verbindungen als wässrige Suspensionen auf die Bodenoberfläche gegossen oder in die Abdeckerde eingearbeitet.

Dabei werden Wirkstoffkonzentrationen von 200 bis 12 ppm (bezogen auf das Bodenvolumen) angewandt. Die Töpfe werden anschließend ca. 3 Wochen in ein Gewächshaus bei 22-24°C gestellt und der Kulturboden gleichmäßig feucht gehalten. Bei der Auswertung auf Phytophthora capsici-Befall werden der Auflauf der Paprika-Pflanzen sowie der Anteil gesunder und kranker Pflanzen im Vergleich zu den entsprechenden Kontrollen ermittelt und entsprechend der Wirkungsgrad errechnet.

Die Verbindungen der Formel I ergeben bei einer Aufwandmenge von 200 ppm eine nahezu vollständige Kontrolle des Phythophtora capsici-Befalls. Eine Reihe von Verbindungen der Formel I führt bei noch wesentlich geringerer Aufwandmenge zu einer ebenfalls nahezu vollständigen Reduktion des Pilzbefalls (0-5 %), wie nachfolgend die in der Tabelle II zusammengefaßten Versuchsergebnisse zeigen.

Tabelle II

Wirkung gegen Phytophthora capsici nach Behandlung von beimpfter Erde mit fungiziden Verbindungen der Formel I und Aussaat von gesundem Paprika-Saatgut.

1. Tag: Beimpfung des Bodens und Behandlung mit fungizider Verbindung, Aussaat.

ca. 21. Tag: Auswertung

| Verbindung gemäß Beispiel Nr. | Wirkungsgrad in % bei ppm Wirkstoff im Boden 200 (ppm) | 100 (ppm) | 50 (ppm) |
|---|---|---|---|
| 1 | 100 | 100 | |
| 9 | 100 | 100 | 100 |
| 113 | 100 | 100 | 100 |
| 3 | 100 | 100 | |
| 2 | 100 | 100 | |
| 122 | 100 | 100 | 100 |
| 120 | 100 | 100 | |
| 21 | 100 | 100 | 100 |
| 115 | 100 | 100 | 100 |
| 8 | 100 | 100 | 100 |
| 23 | 100 | 100 | 100 |
| 139 | 100 | 100 | 100 |
| 121 | 100 | 100 | |
| 123 | 100 | 100 | |
| 148 | 100 | 100 | 100 |
| 129 | 100 | 100 | |
| 134 | 100 | 100 | 100 |
| 14 | 100 | 100 | 100 |
| 16 | 100 | 100 | 100 |
| 36 | 100 | 100 | 100 |
| 68 | 100 | 100 | 100 |
| 4 | 100 | 90 | |
| 28 | 100 | 90 | |
| 25 | 100 | 100 | |
| 27 | 100 | 95 | |
| 86 | 100 | 100 | 100 |
| 130 | 100 | 100 | |
| 171 | 100 | 95 | |
| Kontrolle: | | | |
| unbehandelte, beimpfte Erde | 0 (= 100 % Auflaufschäden) | | |
| unbehandelte, nicht beimpfte Erde | 100 % Auflauf | | |

## Beispiel III

## Wirkung der Verbindungen der Formel I gegen Phytophthora infestans auf Solanum lycopersicum

a) Präventive Wirkung

Tomatenpflanzen (Solanum lycopersicum) der Sorte Rheinlandsruhm werden im 3-Blattstadium mit den beanspruchten Verbindungen der Formel I tropfnaß gespritzt. Die Anwendungskonzentrationen betragen jeweils 500, 250 und 125 mg Wirkstoff pro Liter Spritzbrühe.

Nach dem Antrocknen des Spritzbelages werden die Pflanzen mit einer Zoosporangiensuspension von Phytophthora infestans stark inokuliert und einen Tag lang tropfnaß in eine Klimakammer mit einer Temperatur von 16°C und einer relativen Luftfeuchte von fast 100 % gestellt. Anschließend werden sie in ein Kühlgewächshaus mit einer Temperatur von 15°C und einer relativen Luftfeuchtigkeit von 90 - 95 % gestellt. Nach einer Inkubationszeit von 7 Tagen werden die Pflanzen auf Befall mit Phytophthora untersucht. Letzterer wird in Form von typischen Blattflecken sichtbar, deren Anzahl und Größe als Bewertungsmaßstab für die geprüften Wirkstoffe dienen. Der Befallsgrad wird ausgedrückt in % befallener Blattfläche im Vergleich zu unbehandelten, infizierten Kontrollpflanzen ( = 100 % Befall). Bei diesem Test bewirken die Verbindungen der Formel I bei einer Aufwandmenge von 500 mg/Ltr. Spritzbrühe eine nahezu vollständige Befallsverhinderung. An einigen in der Tabelle III a) zusammengefaßten Versuchsergebnissen kann gezeigt werden, daß viele Verbindungen der Formel I auch bei wesentlich geringeren Aufwandmengen zu einer vollständigen Pilzbefallskontrolle führen.

Tabelle III a

| Verbindung gemäß Beispiel Nr. | % Phytophthora-Befall bei mg Wirkstoff/l Spritzbrühe 500 (mg) | 250 (mg) | 125 (mg) |
|---|---|---|---|
| 113 | 0 | 0 | 0 |
| 3 | 0 | 0 | 0 |
| 2 | 0 | 0 | 0 |
| 122 | 0 | 0 | 0 |
| 120 | 0 | 0 | 0 |
| 21 | 0 | 0 | 0 |
| 115 | 0 | 0 | 0 |
| 134 | 0 | 0 | 0 |
| 148 | 0 | 0 | 0 |
| 8 | 0 | 0 | 0 |
| 14 | 0 | 0 | 0 |
| 16 | 0 | 0 | 0 |
| 17 | 0 | 0 | 2 |
| 36 | 0 | 0 | 0 |
| 81 | 0 | 0 | 0 |
| 86 | 0 | 0 | 3 |
| 194 | 0 | 0 | 0 |
| 4 | 5 | 10 | 25 |
| unbehandelte, infizierte Pflanzen | | 100 | |

b) Curative Wirkung

Tomatenpflanzen (Solanum lycopersicum) der Sorte Rheinlandsruhm werden im 3-Blattstadium mit einer Zoosporangiensuspension von Phytophthora infestans stark inokuliert und 24 Stunden in einer Klimakammer bei einer Temperatur von 15°C und einer relativen Luftfeuchtigkeit von nahezu 100 % inkubiert.

Nach dem Abtrocknen der Pflanzen werden diese mit den

als Spritzpulver formulierten Verbindungen der Formel I bei Wirkstoffkonzentrationen von 500, 250 und 125 mg/Ltr. Spritzbrühe besprüht. Anschließend werden die Pflanzen wieder in ein Gewächshaus mit einer Temperatur von ca. 16 - 18°C und einer relativen Luftfeuchtigkeit von ca. 90 - 95 % zurückgestellt.

Fünf Tage danach werden die Pflanzen auf Befall mit Phytophthora infestans untersucht. Anzahl und Größe der typischen Phytophthora-Blattflecken dienen als Maßstab für die Bewertung der geprüften Wirkstoffe.

Der Befallsgrad wird ausgedrückt in % befallener Blattfläche im Vergleich zu unbehandelten, infizierten Kontrollpflanzen (= 100 % Befall).

Mit den Verbindungen der Formel I wird die Infektion bei einer Aufwandmenge von 500 mg/l Spritzbrühe nahezu vollständig gestoppt. Wie die in der Tabelle III b) zusammengefaßten Versuchsergebnisse zeigen, führen viele Verbindungen der Formel I bei noch wesentlich geringeren Aufwandmengen zu einer vollständigen Befallskontrolle.

Tabelle III b

| Verbindung gemäß Beispiel Nr. | % Phytophthora-Befall bei mg Wirkstoff/l Spritzbrühe | | |
|---|---|---|---|
| | 500 (mg) | 250 (mg) | 125 (mg) |
| 113 | 0 | 0 | 0-3 |
| 3 | 0 | 0 | 0-3 |
| 2 | 0 | 0 | 0 |
| 122 | 0 | 0 | 3 |
| 120 | 0 | 0 | 0 |
| 21 | 0 | 0 | 0 |
| 115 | 0 | 0 | 0 |
| unbehandelte, infizierte Pflanzen | | 100 | |

c) Präventiv-systemische Wirkung

Die als Spritzpulver formulierten Verbindungen der Formel I werden als wässrige Suspensionen in Wirkstoffkonzentrationen von 200, 100, 50 und 25 ppm (bezogen auf das Bodenvolumen) auf die Bodenoberfläche von ca. 3-4 Wochen alten, eingetopften Tomatenpflanzen der Sorte Rheinlandsruhm gegeben. Drei Tage später werden die Pflanzen mit einer Zoosporangiensuspension von Phytophthora infestans inokuliert und wie in dem Beispiel III a) beschrieben, inkubiert. Nach einer Inkubationszeit von 6-7 Tagen werden die Pflanzen auf Befall mit Phytophthora infestans untersucht. Anzahl und Größe der auf den Blättern gebildeten, typischen Phytophthora-Blattflecken bilden den Maßstab für die Beurteilung der Wirksamkeit der geprüften Verbindungen.

Der Befallsgrad wird ausgedrückt in % befallener Blattfläche im Vergleich zu unbehandelten, infizierten Kontrollpflanzen (= 100 % Befall).

Mit den Verbindungen der Formel I wird der Befall bei einer Aufwandmenge von 200 ppm Wirkstoff (bezogen auf das Bodenvolumen) nahezu vollständig verhindert. Wie die in der Tabelle III c) zusammengefaßten Versuchsergebnisse zeigen, führen viele Verbindungen der Formel I bei noch wesentlich geringeren Aufwandmengen zu einer vollständigen Befallskontrolle.

Tabelle III c

| Verbindung gemäß Beispiel Nr. | % Phytophthora-Befall bei ppm Wirkstoff im Boden 200 (ppm) | 100 (ppm) | 50 (ppm) |
|---|---|---|---|
| 113 | 0 | 0 | 0 |
| 121 | 0 | 0 | 0 |
| 134 | 0 | 0 | 0 |
| 148 | 0 | 0 | 0 |
| 149 | 0 | 0 | 0 |
| 8 | 0 | 0 | 0 |
| 10 | 0 | 0 | 0 |
| 14 | 0 | 0 | 0 |
| 16 | 0 | 0 | 0 |
| 36 | 0 | 0 | 0 |
| 37 | 0 | 0 | 0 |
| 194 | 0 | 0 | 0 |
| infizierte Pflanzen, unbehandelter Boden | | | 100 |

Beispiel IV

Wirkung der Verbindungen der Formel I gegen Plasmopara viticola auf Reben

Präventive Wirkung

Weinpflanzen, die aus Stecklingen der Plasmoparaanfälligen Sorte Müller-Thurgau gezogen waren, werden im 4-Blattstadium mit wässrigen Suspensionen der zu prüfenden Verbindungen der Formel I tropfnaß gespritzt. Die Anwendungskonzentrationen betragen 500, 250 und 125 mg Wirkstoff/l Spritzbrühe.

Nach dem Antrocknen des Spritzbelages werden die Pflanzen mit einer Zoosporangiensuspension von Plasmopara viticola inokuliert und tropfnaß in eine Klimakammer mit einer Temperatur von 20°C und einer relativen Luftfeuchte von ca. 100 % gestellt. Nach 24 Stunden werden die infizierten Pflanzen der Klimakammer entnommen und in ein Gewächshaus mit einer Temperatur von 23°C und einer Luftfeuchtigkeit von ca. 98 % gebracht. Nach einer Inkubationszeit von 7 Tagen werden die Pflanzen angefeuchtet, über Nacht in die Klimakammer gestellt und die Krankheit zum Ausbruch gebracht. Anschließend erfolgt die Befallsauswertung. Anzahl und Größe der Infektionsstellen auf den Blättern der inokulierten und behandelten Pflanzen dienen als Maßstab für die Wirksamkeit der beanspruchten Verbindungen. Der Befallsgrad wird ausgedrückt in % befallener Blattfläche im Vergleich zu unbehandelten, infizierten Kontrollpflanzen (= 100 % Befall).

Die Verbindungen der Formel I geben bei einer Aufwandmenge von 500 mg Wirkstoff/Ltr. Spritzbrühe eine durchschnittliche Befallskontrolle von $>$ 80 %. Wie an einigen in der Tabelle IV zusammengefaßten Versuchsergebnissen gezeigt werden kann, können viele Verbindungen der Formel I auch bei wesentlich geringeren Aufwandmengen zu einer vollständigen Pilzbefallskontrolle führen.

Tabelle IV

| Verbindung gemäß Beispiel Nr. | % Plasmopara-Befall bei mg Wirkstoff/l Spritzbrühe 500 (mg) | 250 (mg) | 125 (mg) |
|---|---|---|---|
| 113 | 0 | 0 | 0 |
| 3 | 0 | 0 | 0 |
| 2 | 0 | 0 | 0 |
| 122 | 0 | 0 | 0 |
| 120 | 0 | 0 | 0 |
| 21 | 0 | 0 | 0 |
| 115 | 0 | 0 | 0 |
| 8 | 0 | 0 | 0 |
| 134 | 0 | 0 | 0 |
| 136 | 0 | 0 | 0 |
| 171 | 0 | 0 | 0 |
| 121 | 0 | 0 | 0 |
| 123 | 0 | 0 | 0 |
| 116 | 0 | 0 | 0 |
| 148 | 0 | 0 | 0 |
| 149 | 0 | 0 | 0 |
| 185 | 0 | 0 | 0 |
| 139 | 0 | 0 | 0 |
| 10 | 0 | 0 | 0 |
| 14 | 0 | 0 | 0 |
| 36 | 0 | 0 | 0 |
| 37 | 0 | 0 | 0 |
| 54 | 0 | 3 | |
| 171 | 0 | 3 | 5 - 10 |
| 73 | 0 | 0 | 0 |
| 185 | 0 | 0 | 0 - 3 |
| 86 | 0 | 0 | 0 |
| unbehandelte, infizierte Pflanzen | | 100 | |

PATENTANSPRÜCHE:

1. Verbindungen der allgemeinen Formel I,

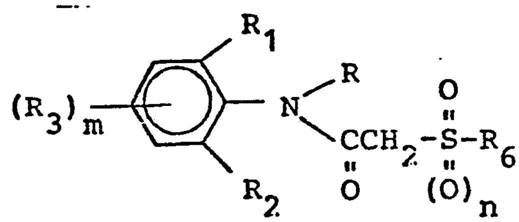

(I)

worin

R = 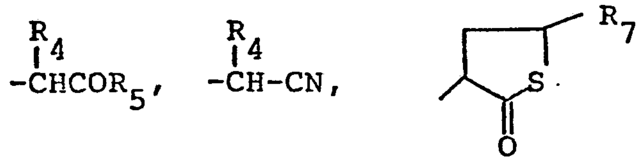 ,

$R_1$ = $(C_1-C_4)$-Alkyl, Halogen, $(C_1-C_4)$-Alkoxy, $(C_1-C_4)$-Alkylthio, $(C_2-C_4)$-Alkenyl,

$R_2$ = Wasserstoff, $(C_1-C_4)$-Alkyl, Halogen,

$R_3$ = $(C_1-C_4)$-Alkyl, Halogen,

$R_4$, $R_7$ = Wasserstoff, Methyl,

$R_5$ = Hydroxy, $(C_1-C_4)$-Alkoxy, $(C_1-C_3)$-Alkylthio, $(C_1-C_2)$-Alkoxyäthoxy, Amino, $(C_1-C_2)$-Alkylamino, Di-$(C_1-C_2)$-alkylamino, O-Kat, wobei Kat für ein Kationäquivalent einer anorganischen oder organischen Base steht,

$R_6$ = $(C_1-C_8)$-Alkyl, $(C_1-C_4)$-Alkoxy-$(C_1-C_3)$-alkyl, $(C_1-C_2)$-Alkylthio-$(C_1-C_3)$-alkyl, $(C_1-C_2)$-Alkylsulfinyl-$(C_1-C_3)$-alkyl, $(C_1-C_2)$-Alkylsulfonyl-$(C_1-C_3)$-alkyl, $(C_3-C_6)$-Cycloalkyl, $(C_1-C_4)$-Halogenalkyl, $(C_1-C_4)$-Hydroxyalkyl, $(C_1-C_2)$-Alkoxycarbonyl-$(C_1-C_2)$-alkyl, $(C_3-C_4)$-Alkenyl, $(C_3-C_4)$-Alkinyl, Benzyl, das gegebenenfalls substituiert ist durch ein oder zwei Reste aus der Gruppe $(C_1-C_4)$-Alkyl, Halogen, Nitro; Phenyl, das gegebenenfalls

substituiert ist durch ein oder zwei Reste aus der Gruppe $(C_1-C_4)$-Alkyl, Halogen,

n = 0, 1 und

m = 0, 1, 2

bedeutet.

2. Verfahren zur Herstellung von Verbindungen der Formel I gemäß Anspruch 1, dadurch gekennzeichnet,

a) daß man Verbindungen der allgemeinen Formel III,

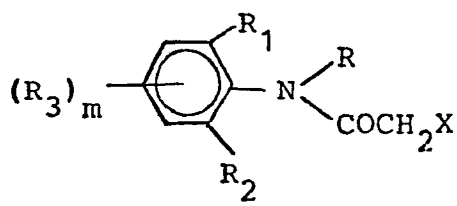

(III)

worin R, $R_1$, $R_2$, $R_3$ und m die Bedeutungen wie in Formel I haben und X einen nukleophil substituierbaren Abgangsrest, vorzugsweise aus der Gruppe Halogen, Tosylat oder Mesylat, insbesondere Chlor oder Brom, bedeutet, mit sulfinsauren Salzen der allgemeinen Formel II,

$$Me - SO_2 - R_6 \qquad \text{(II)}$$

in der Me ein anorganisches oder organisches Kation, vorzugsweise Natrium, Kalium, Calcium oder Ammonium, bedeutet und $R_6$ die Bedeutung wie in Formel I hat, zu Verbindungen der Formel I, in der n=1 bedeutet, umsetzt, vorzugsweise in inerten Lösungs- oder Verdünnungsmitteln und bevorzugt bei Temperaturen zwischen 50 und 250°C,
oder

b) daß man Anilinderivate der allgemeinen Formel IV,

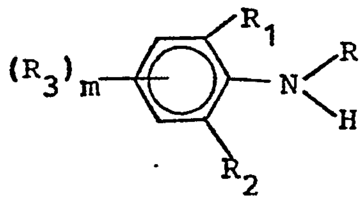

(IV)

worin R, $R_1$, $R_2$, $R_3$ und m die Bedeutungen wie in Formel I haben, entweder mit Sulfonylacetylhalogeniden der allgemeinen Formel V,

$$R_6 - SO_2 - CH_2 - COHal \qquad (V)$$

worin Hal ein Halogenatom, vorzugsweise Chlor oder Brom, bedeutet und $R_6$ die Bedeutung wie in Formel I hat, zu Verbindungen der Formel I, in der n=1 bedeutet, oder mit Sulfinylacetylhalogeniden der allgemeinen Formel VII,

$$R_6 - SO - CH_2 - COHal \qquad (VII)$$

in der $R_6$ und Hal die Bedeutungen wie in Formel V haben, zu Verbindungen der Formel I, in der n=0 bedeutet, umsetzt, gegebenenfalls jeweils unter Zusatz von säurebindenden Mitteln, vorzugsweise jeweils in inerten Lösungs- oder Verdünnungsmitteln und bevorzugt jeweils bei Temperaturen zwischen -20 und 160°C,

oder

c) daß man Mercaptoacetanilide der allgemeinen Formel VI,

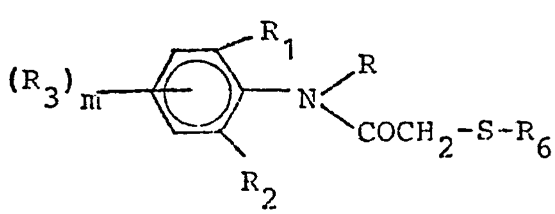

(VI)

worin R, $R_1$, $R_2$, $R_3$, $R_6$ und m die Bedeutungen wie in Formel I haben, mit entsprechenden Mengen eines Oxidationsmittels entweder zu Verbindungen der Formel I, in der n=O bedeutet, oder zu Verbindungen der Formel I, in der n=1 bedeutet, umsetzt, vorzugsweise jeweils in Lösungs- oder Verdünnungsmitteln und bevorzugt bei Temperaturen zwischen -40 und 100°C für Verbindungen der Formel I mit n=O, bzw. zwischen -20 und 160°C für Verbindungen der Formel I mit n=1.

3. Fungizide Mittel, gekennzeichnet durch einen Gehalt an einer Verbindung der Formel I gemäß Anspruch 1 als Wirkstoff.

4. Fungizide Mittel, gekennzeichnet durch einen Gehalt von 2 bis 95 Gew.-%, vorzugsweise 10 bis 90 Gew.-% an einer Verbindung der Formel I gemäß Anspruch 1 als Wirkstoff sowie üblichen Formulierungshilfsmitteln.

5. Verwendung von Verbindungen der Formel I gemäß Ansprüchen 1, 3 und 4 zur Schädlingsbekämpfung im Pflanzenschutz.

6. Verfahren zur Bekämpfung von Schadpilzen, dadurch gekennzeichnet, daß man die von ihnen befallenen Flächen, Pflanzen oder Substrate mit einer fungizid wirksamen Menge von Wirkstoffen der Formel I gemäß Ansprüchen 1, 3 und 4 in Kontakt bringt.

Patentansprüche für Österreich

1. Verfahren zur Herstellung von Verbindungen der allgemeinen Formel I

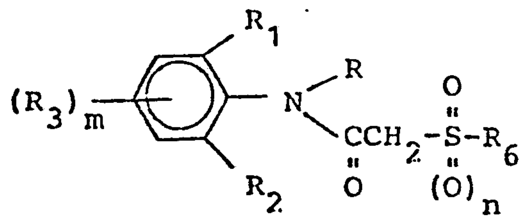

(I)

worin

| | | |
|---|---|---|
| R | = | $-\overset{R_4}{\overset{\vert}{C}}HCOR_5$, $-\overset{R_4}{\overset{\vert}{C}}H-CN$, 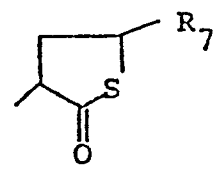 , |
| $R_1$ | = | $(C_1-C_4)$-Alkyl, Halogen, $(C_1-C_4)$-Alkoxy, $(C_1-C_4)$-Alkylthio, $(C_2-C_4)$-Alkenyl, |
| $R_2$ | = | Wasserstoff, $(C_1-C_4)$-Alkyl, Halogen, |
| $R_3$ | = | $(C_1-C_4)$-Alkyl, Halogen, |
| $R_4$, $R_7$ | = | Wasserstoff, Methyl, |
| $R_5$ | = | Hydroxy, $(C_1-C_4)$-Alkoxy, $(C_1-C_3)$-Alkylthio, $(C_1-C_2)$-Alkoxyäthoxy, Amino, $(C_1-C_2)$-Alkylamino, Di-$(C_1-C_2)$-alkylamino, O-Kat, wobei Kat für ein Kationäquivalent einer anorganischen oder organischen Base steht, |
| $R_6$ | = | $(C_1-C_8)$-Alkyl, $(C_1-C_4)$-Alkoxy-$(C_1-C_3)$-alkyl, $(C_1-C_2)$-Alkylthio-$(C_1-C_3)$-alkyl, $(C_1-C_2)$-Alkylsulfinyl-$(C_1-C_3)$-alkyl, $(C_1-C_2)$-Alkylsulfonyl-$(C_1-C_3)$-alkyl, $(C_3-C_6)$-Cycloalkyl, $(C_1-C_4)$-Halogenalkyl, $(C_1-C_4)$-Hydroxyalkyl, $(C_1-C_2)$-Alkoxycarbonyl-$(C_1-C_2)$-alkyl, $(C_3-C_4)$-Alkenyl, $(C_3-C_4)$-Alkinyl, Benzyl, das gegebenenfalls substituiert ist durch ein oder zwei Reste aus der Gruppe $(C_1-C_4)$-Alkyl, Halogen, Nitro; Phenyl, das gegebenenfalls |

BAD ORIGINAL

substituiert ist durch ein oder zwei Reste aus der Gruppe $(C_1-C_4)$-Alkyl, Halogen,

n = 0, 1 und

m = 0, 1, 2

bedeutet, dadurch gekennzeichnet,

a) daß man Verbindungen der allgemeinen Formel III,

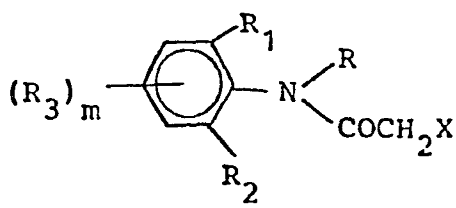

(III)

worin R, $R_1$, $R_2$, $R_3$ und m die Bedeutungen wie in Formel I haben und X einen nukleophil substituierbaren Abgangsrest, vorzugsweise aus der Gruppe Halogen, Tosylat oder Mesylat, insbesondere Chlor oder Brom, bedeutet, mit sulfinsauren Salzen der allgemeinen Formel II,

$$Me - SO_2 - R_6 \qquad (II)$$

in der Me ein anorganisches oder organisches Kation, vorzugsweise Natrium, Kalium, Calcium oder Ammonium, bedeutet und $R_6$ die Bedeutung wie in Formel I hat, zu Verbindungen der Formel I, in der n=1 bedeutet, umsetzt, vorzugsweise in inerten Lösungs- oder Verdünnungsmitteln und bevorzugt bei Temperaturen zwischen 50 und 250°C,
oder

b) daß man Anilinderivate der allgemeinen Formel IV,

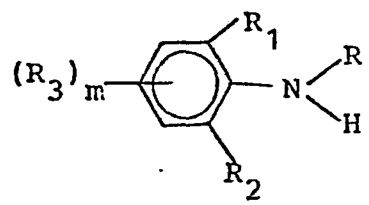

(IV)

worin R, $R_1$, $R_2$, $R_3$ und m die Bedeutungen wie in Formel I haben, entweder mit Sulfonylacetylhalogeniden der allgemeinen Formel V,

$$R_6 - SO_2 - CH_2 - COHal \qquad (V)$$

worin Hal ein Halogenatom, vorzugsweise Chlor oder Brom, bedeutet und $R_6$ die Bedeutung wie in Formel I hat, zu Verbindungen der Formel I, in der n=1 bedeutet, oder mit Sulfinylacetylhalogeniden der allgemeinen Formel VII,

$$R_6 - SO - CH_2 - COHal \qquad (VII)$$

in der $R_6$ und Hal die Bedeutungen wie in Formel V haben, zu Verbindungen der Formel I, in der n=0 bedeutet, umsetzt, gegebenenfalls jeweils unter Zusatz von säurebindenden Mitteln, vorzugsweise jeweils in inerten Lösungs- oder Verdünnungsmitteln und bevorzugt jeweils bei Temperaturen zwischen -20 und 160°C,
oder

c) daß man Mercaptoacetanilide der allgemeinen Formel VI,

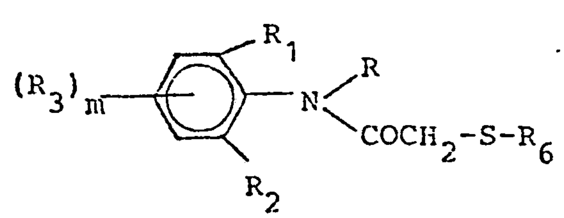

(VI)

worin R, $R_1$, $R_2$, $R_3$, $R_6$ und m die Bedeutungen wie in Formel I haben, mit entsprechenden Mengen eines Oxidationsmittels entweder zu Verbindungen der Formel I, in der n=0 bedeutet, oder zu Verbindungen der Formel I, in der n=1 bedeutet, umsetzt, vorzugsweise jeweils in Lösungs- oder Verdünnungsmitteln und bevorzugt bei Temperaturen zwischen -40 und 100°C für Verbindungen der Formel I mit n=0, bzw. zwischen -20 und 160°C für Verbindungen der Formel I mit n=1.

2. Fungizide Mittel, gekennzeichnet durch einen Gehalt an einer Verbindung der Formel I gemäß Anspruch 1 als Wirkstoff.

3. Fungizide Mittel, gekennzeichnet durch einen Gehalt von 2 bis 95 Gew.-%, vorzugsweise 10 bis 90 Gew.-% an einer Verbindung der Formel I gemäß Anspruch 1 als Wirkstoff sowie üblichen Formulierungshilfsmitteln.

4. Verwendung von Verbindungen der Formel I gemäß Ansprüchen 1, 2 und 3 zur Schädlingsbekämpfung im Pflanzenschutz.

5. Verfahren zur Bekämpfung von Schadpilzen, dadurch gekennzeichnet, daß man die von ihnen befallenen Flächen, Pflanzen oder Substrate mit einer fungizid wirksamen Menge von Wirkstoffen der Formel I gemäß Ansprüchen 1, 2 und 3 behandelt.

0034205

Europäisches Patentamt

# EUROPÄISCHER RECHERCHENBERICHT

Nummer der Anmeldung

EP 80 10 6946

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | FR - A - 2 407 210 (CHEVRON)<br>* Ansprüche *<br>& DE - A - 2 845 454<br>-- | 1,3-6 | C 07 C 147/00<br>C 07 D 333/36<br>A 01 N 41/10<br>43/10 |
| D | FR - A - 2 267 042 (CIBA-GEIGY)<br>* Ansprüche *<br>& DE - A - 2 515 901<br>-- | 1-6 | |
| PX | FR - A - 2 443 454 (CIBA-GEIGY)<br>-- | | RECHERCHIERTE SACHGEBIETE (Int. Cl.³)<br>C 07 C 147/00<br>C 07 D 333/36<br>A 01 N 41/10<br>43/10 |
| A | CHEMICAL ABSTRACTS, Band 87, Nr. 23, 5. Dezember 1977, Seite 9, Zusammenfassung Nr. 177392b, Columbus, Ohio, US,<br>J.E. BAKKE et al.: "Replacement of a chlorine with a methylsulfonyl group in the metabolism of propachlor (2-chloro-N-isopropylacetanilide"<br>& BIOMED. MASS SPECTROM., 1976, 3(5), 226-9<br>& CHEMICAL ABSTRACTS, Formula Index, A-$C_{15}$, Juli-Dezember 1977 Seite 1103<br>* Seite 1103, Mittelspalte *<br>-- | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| PX | FR - A - 2 443 454 (CIBA-GEIGY)<br>* Ansprüche *<br>& DE - A - 2 948 568<br>----- | 1,3-6 | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 23-02-1981 | MOREAU |